# EUROPEAN PATENT SPECIFICATION

(11) **EP 4 243 149 B1**
(45) Date of publication and mention of the grant of the patent: **28.01.2026**
(21) Application number: 22895872.4
(22) Date of filing: 21.10.2022
(51) Int. Cl.: H01M 10/0567, H01M 10/052, H01M 4/525, H01M 4/505, H01M 4/36, H01M 4/587, H01M 4/38, H01M 4/48, H01M 4/58, C07C 311/48, H01M 10/0525

(54) **NOVEL ADDITIVE FOR NONAQUEOUS ELECTROLYTE, AND LITHIUM SECONDARY BATTERY COMPRISING SAME**
NEUARTIGES ADDITIV FÜR WASSERFREIEN ELEKTROLYT UND LITHIUMSEKUNDÄRBATTERIE DAMIT
NOUVEL ADDITIF POUR ÉLECTROLYTE NON AQUEUX, ET BATTERIE SECONDAIRE AU LITHIUM LE COMPRENANT

(30) Priority: 16.11.2021 KR 20210157225; 13.10.2022 KR 20220131377
(43) Date of publication of application: 13.09.2023
(73) Proprietor: LG Energy Solution, Ltd., Seoul 07335 (KR)
(72) Inventor: JEONG, You Kyeong, Daejeon 34122 (KR); AHN, Kyoung Ho, Daejeon 34122 (KR); HAN, Jun Hyeok, Daejeon 34122 (KR); SHIN, Won Kyung, Daejeon 34122 (KR); LEE, Won Tae, Daejeon 34122 (KR); JI, Su Hyeon, Daejeon 34122 (KR); OH, Young Ho, Daejeon 34122 (KR)
(74) Representative: Hoffmann Eitle
(86) International application number: PCT/KR2022/016109
(87) International publication number: WO 2023/090664

(56) References cited:
- WO-A1-2012/042005
- JP-A- 2000 082 494
- JP-A- 2010 225 510
- KR-A- 20170 021 335
- US-A1- 2014 272 600
- US-A1- 2017 058 066
- US-A1- 2021 257 659

## Description

### [Technical Field]

The present disclosure relates to a novel additive for a non-aqueous electrolyte and a lithium secondary battery including the same.

This application claims priority from Korean Patent Application No. 10-2021-0157225, filed on November 16, 2021, and Korean Patent Application No. 10-2022-0131377, filed on October 13, 2022.

### [Background]

Recently, secondary batteries are widely applied not only to small devices such as portable electronic devices, but to medium and large devices such as battery packs or power storage devices of hybrid or electric vehicles. Examples of these secondary batteries may include non-aqueous electrolyte batteries such as lithium-ion batteries, lithium batteries, lithium-ion capacitors, and sodium ion batteries.

Among these non-aqueous electrolyte batteries, lithium-ion batteries are used by injecting an electrolyte into a battery cell including a positive electrode including a positive electrode active material that enables intercalation and deintercalation of lithium and a negative electrode including a negative electrode active material that enables intercalation and deintercalation of lithium. Particularly, an electrolyte uses an organic solvent in which a lithium salt is dissolved, and it is important to determine the stability and performance of a lithium secondary battery.

For example, LiPF₆, which is the most widely used lithium salt for an electrolyte, reacts with an electrolyte solvent to promote the depletion of the solvent and generate HF. The HF generated thereby may not only generate a large amount of gas under a high temperature condition, but also elute metal ions from a positive electrode active material, and when the eluted metal ions are generated in the form of a precipitate on the surface of a negative electrode, it causes an increase in potential of a negative electrode and a drop in cell open-circuit voltage (OCV), leading to problems such as degraded battery performance as well as a reduction in lifespan and high-temperature safety.

US 2021/257659 describes a composition for a gel polymer electrolyte and a lithium secondary battery including a gel polymer electrolyte formed therefrom.

JP2000 082494 describes a flame-retardant non-aqueous electrolyte used for electrochemical devices such as secondary batteries, primary batteries, and electric double layer capacitors, and is particularly suitable for secondary batteries.

JP2010 22 5510 describes an electrolyte composition and a secondary battery, and more particularly an electrolyte composition that is flame retardant.

### [Description of the Invention]

### [Technical Problem]

Therefore, the present disclosure is directed to providing a developed technology of forming a coating film on an electrode surface to prevent direct contact between a positive electrode and an electrolyte, inhibit gas generation by lowering the oxidative decomposition of the electrolyte while blocking direct contact between the positive electrode and HF or PF₅, and improve a capacity retention rate and prevent the OCV drop of a battery by improving a metal ion precipitation phenomenon of the positive electrode.

### [Technical Solution]

To solve the above-described problem, the present invention is as set out in the attached claims.
one embodiment of the present disclosure provides
a lithium secondary battery, comprising:
   an electrode assembly having a positive electrode, a negative electrode, and a separator interposed between the positive electrode and the negative electrode, and
   an electrolyte composition for a lithium secondary battery,
   wherein the positive electrode comprises one or more positive electrode active materials of lithium metal oxides represented by Formulas 2 and 3:

      [Formula 2] Liₓ[Ni_{y}Co_{z}Mn_{w}M¹ᵥ]O₂

      [Formula 3] LiM²ₚMn₍₂₋ₚ₎O₄

In Formulas 2 and 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6, wherein the electrolyte composition for a lithium secondary battery comprises a non-aqueous organic solvent;
a lithium salt; and
a compound represented by Formula 1 below: 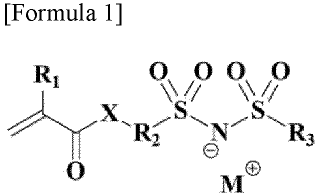
wherein,
R₁ is hydrogen or an alkyl group having 1 to 4 carbon atoms,
R₂ includes one or more selected from an alkylene group having 1 to 10 carbon atoms, an oxyalkylene group having 1 to 10 carbon atoms, a cycloalkylene group having 5 to 10 carbon atoms, and 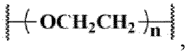
R₃ is a fluoro group, an alkyl group having 1 to 10 carbon atoms, an alkoxy group having 1 to 10 carbon atoms, or 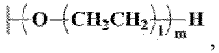
wherein one or more hydrogens included in the alkyl groups, the alkylene group, the alkoxy group, the oxyalkylene group, the cycloalkylene group, 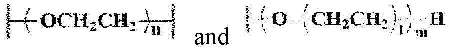 are optionally substituted with a fluorine atom,
X is an oxygen atom (O) or -NR₄, wherein R₄ is hydrogen or an alkyl group having 1 to 4 carbon atoms,
M includes one or more selected from the group consisting of lithium, sodium, potassium, tetraalkyl ammonium having 1 to 4 carbon atoms, and tetraalkyl phosphonium having 1 to 4 carbons,
1 is an integer of 1 to 6, and
each of m and n is an integer of 2 to 20.

Specifically, R₁ may be hydrogen or a methyl group,
R₂ comprises one or more selected from a methylene group, an ethylene group, a propylene group, an oxymethylene group, an oxyethylene group, an oxypropylene group, a cyclobutylene group, a cyclopentylene group, a cyclohexylene group, a cycloheptylene group, 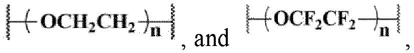
R₃ may be a fluoro group, a methyl group, an ethyl group, a propyl group, a methoxy group, an ethoxy group, 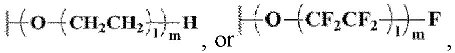
X may be an oxygen atom (O), -NH, or -NCH₃, M may be lithium, 1 may be an integer of 1 or 2, and each of m and n may be an integer of 2 to 10.

More specifically, the compound represented by Formula 1 may be one or more compounds selected from Structural Formula 1 to Structural Formula 120 below:

| | | |
|---|---|---|
| Structural Formula 1 | Structural Formula 2 | Structural Formula 3 |
| 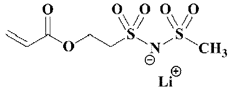 | 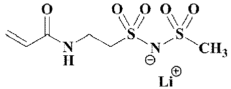 | 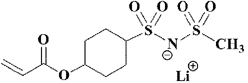 |
| Structural Formula 4 | Structural Formula 5 | Structural Formula 6 |
| 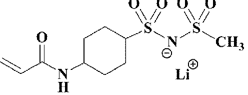 | 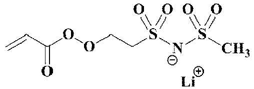 | 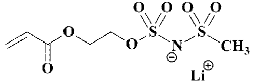 |
| Structural Formula 7 | Structural Formula 8 | Structural Formula 9 |
| 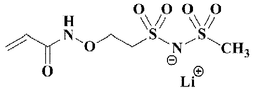 | 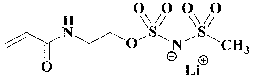 | 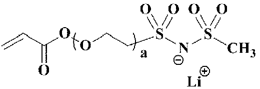 |
| Structural Formula 10 | Structural Formula 11 | Structural Formula 12 |
| 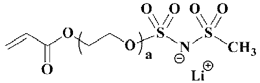 | 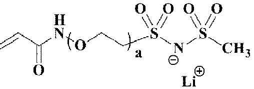 | 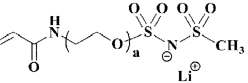 |

| | | |
|---|---|---|
| | Structural Formula 14 | Structural Formula 15 |
| | 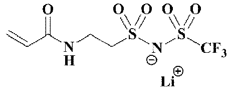 | 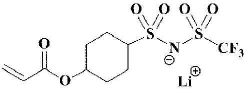 |
| Structural Formula 16 | Structural Formula 17 | Structural Formula 18 |
| 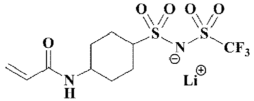 | 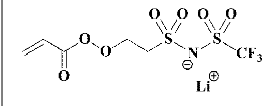 | 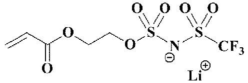 |
| Structural Formula 19 | Structural Formula 20 | Structural Formula 21 |
| 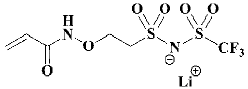 | 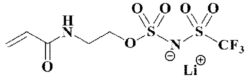 | 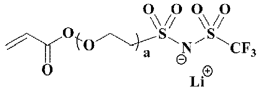 |
| Structural Formula 22 | Structural Formula 23 | Structural Formula 24 |
| 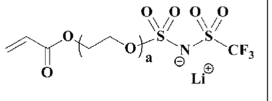 | 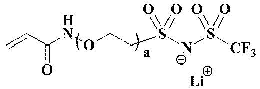 | 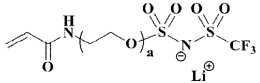 |

| | | |
|---|---|---|
| Structural Formula 25 | Structural Formula 26 | Structural Formula 27 |
| 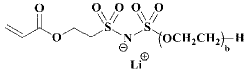 | 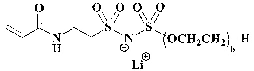 | 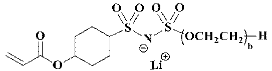 |
| Structural Formula 28 | Structural Formula 29 | Structural Formula 30 |
| 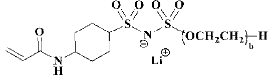 | 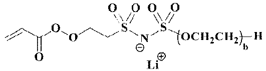 | 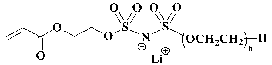 |
| Structural Formula 31 | Structural Formula 32 | Structural Formula 33 |
| 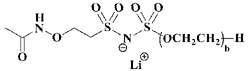 | 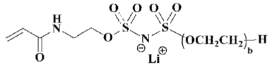 | 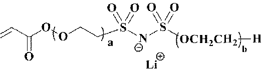 |
| Structural Formula 34 | Structural Formula 35 | Structural Formula 36 |
| 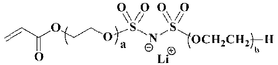 | 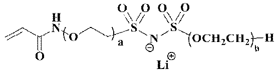 | 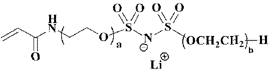 |

| | | |
|---|---|---|
| Structural Formula 37 | Structural Formula 38 | Structural Formula 39 |
| 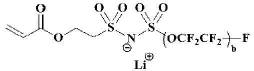 | 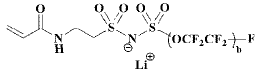 | 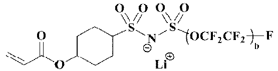 |
| Structural Formula 40 | Structural Formula 41 | Structural Formula 42 |
| 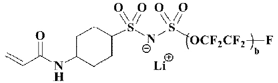 | 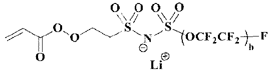 | 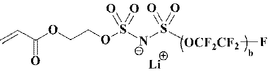 |
| Structural Formula 43 | Structural Formula 44 | Structural Formula 45 |
| 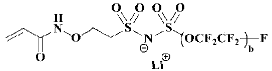 | 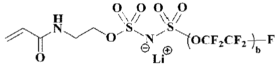 | 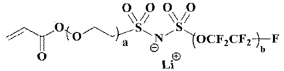 |
| Structural Formula 46 | Structural Formula 47 | Structural Formula 48 |
| 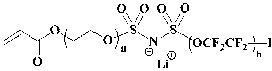 | 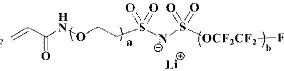 | 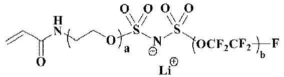 |

| | | |
|---|---|---|
| Structural Formula 49 | Structural Formula 50 | Structural Formula 51 |
| 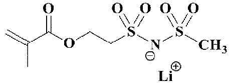 | 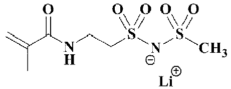 | 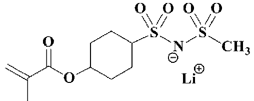 |
| Structural Formula 52 | Structural Formula 53 | Structural Formula 54 |
| 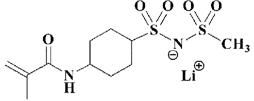 | 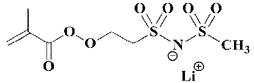 | 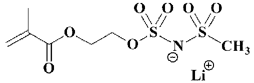 |
| Structural Formula 55 | Structural Formula 56 | Structural Formula 57 |
| 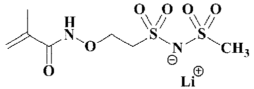 | 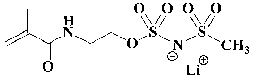 | 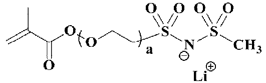 |
| Structural Formula 58 | Structural Formula 59 | Structural Formula 60 |
| 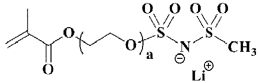 | 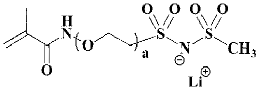 | 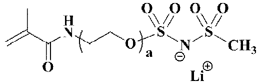 |

| | | |
|---|---|---|
| | Structural Formula 62 | Structural Formula 63 |
| | 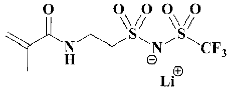 | 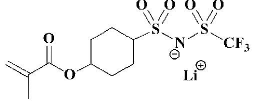 |
| Structural Formula 64 | Structural Formula 65 | Structural Formula 66 |
| 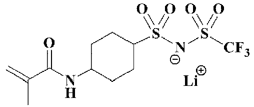 | 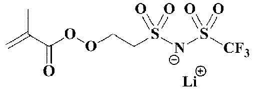 | 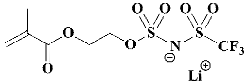 |
| Structural Formula 67 | Structural Formula 68 | Structural Formula 69 |
| 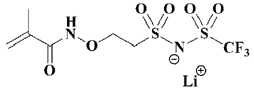 | 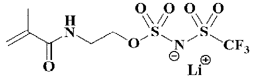 | 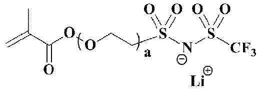 |
| Structural Formula 70 | Structural Formula 71 | Structural Formula 72 |
| 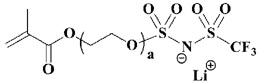 | 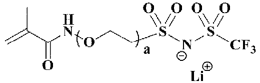 | 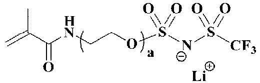 |

| | | |
|---|---|---|
| Structural Formula 73 | Structural Formula 74 | Structural Formula 75 |
| 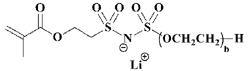 | 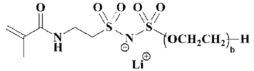 | 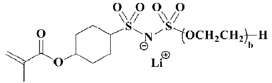 |
| Structural Formula 76 | Structural Formula 77 | Structural Formula 78 |
| 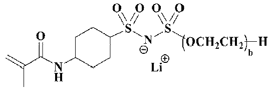 | 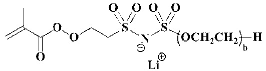 | 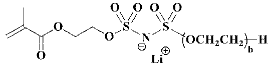 |
| Structural Formula 79 | Structural Formula 80 | Structural Formula 81 |
| 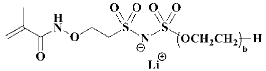 | 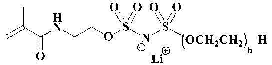 | 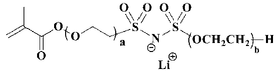 |
| Structural Formula 82 | Structural Formula 83 | Structural Formula 84 |
| 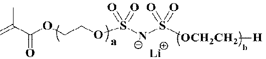 | 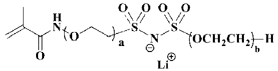 | 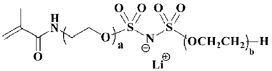 |

| | | |
|---|---|---|
| Structural Formula 85 | Structural Formula 86 | Structural Formula 87 |
| 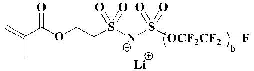 | 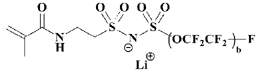 | 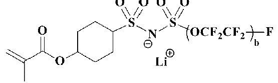 |
| Structural Formula 88 | Structural Formula 89 | Structural Formula 90 |
| 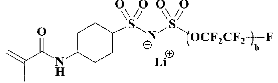 | 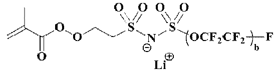 | 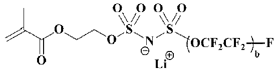 |
| Structural Formula 91 | Structural Formula 92 | Structural Formula 93 |
| 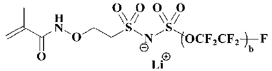 | 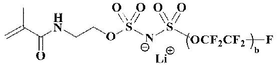 | 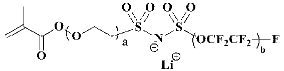 |
| Structural Formula 94 | Structural Formula 95 | Structural Formula 96 |
| 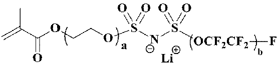 | 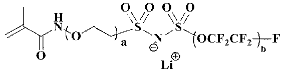 | 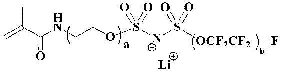 |

| | | |
|---|---|---|
| Structural Formula 97 | Structural Formula 98 | Structural Formula 99 |
| 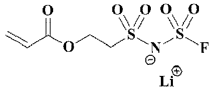 | 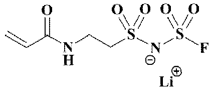 | 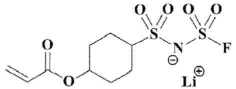 |
| Structural Formula 100 | Structural Formula 101 | Structural Formula 102 |
| 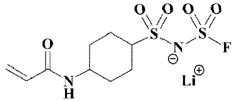 | 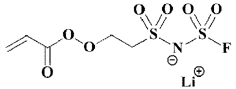 | 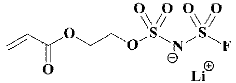 |
| Structural Formula 103 | Structural Formula 104 | Structural Formula 105 |
| 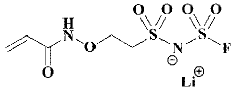 | 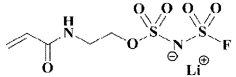 | 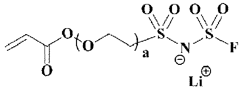 |
| Structural Formula 106 | Structural Formula 107 | Structural Formula 108 |
| 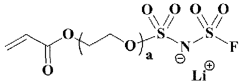 | 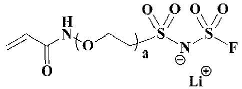 | 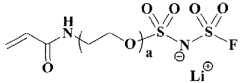 |

| | | |
|---|---|---|
| Structural Formula 109 | Structural Formula 110 | Structural Formula 111 |
| 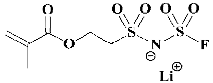 | 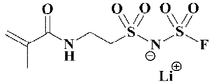 | 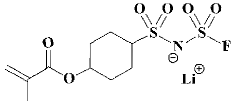 |
| Structural Formula 112 | Structural Formula 113 | Structural Formula 114 |
| 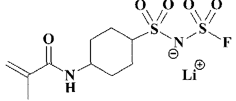 | 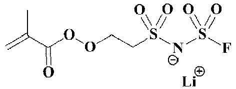 | 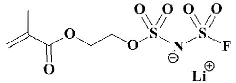 |
| Structural Formula 115 | Structural Formula 116 | Structural Formula 117 |
| 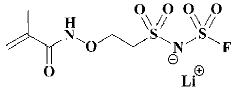 | 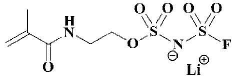 | 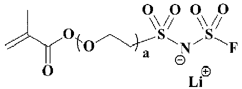 |
| Structural Formula 118 | Structural Formula 119 | Structural Formula 120 |
| 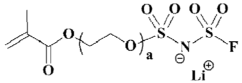 | 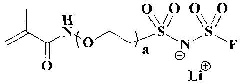 | 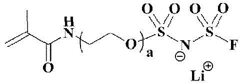 |

In Structural Formula 9~12, 21~48, 57~60, 69~96, 105~108, and 117~120 a and b is an integer of 2 to 4 respectively.

In addition, one embodiment of the present disclosure provides
an electrolyte composition for a lithium secondary battery, which includes a non-aqueous organic solvent; a lithium salt; and a compound represented by Formula 1, wherein the compound represented by Formula 1 is one or more compounds selected from Structural Formula 1 to Structural Formula 120, reported in the tables above (compounds 13 and 61 are excluded):

Here, the compound represented by Formula 1 may be included at 0.01 to 3 wt% with respect to the total weight of the electrolyte composition.

In addition, the lithium salt may include one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

In addition, the non-aqueous organic solvent may include N-methyl-2-pyrrolidinone, ethylene carbonate, propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, a dioxolane derivative, sulfolane, methyl sulfolane, 1,3-dimethyl-2-imidazolidinone, a propylene carbonate derivative, a tetrahydrofuran derivative, ether, methyl propionate, and ethyl propionate.

Furthermore, one embodiment of the present disclosure provides a lithium secondary battery, which includes:
an electrode assembly that includes a positive electrode including one or more positive electrode active materials of lithium metal oxides represented by Formulas 2 and 3 below, a negative electrode, and a separator interposed between the positive electrode and the negative electrode; and
the electrolyte composition according to the present disclosure:

   [Formula 2] Liₓ[Ni_{y}Co_{z}Mn_{w}M¹ᵥ]O₂

   [Formula 3] LiM²ₚMn₍₂₋ₚ₎O₄

In Formulas 2 and 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6.

Here, the positive electrode active material may include one or more selected from the group consisting of LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂, and LiNi_{0.5}Mn_{1.5}O₄.

In addition, the negative electrode active material may consist of a carbon material and a silicon material, wherein the silicon material may include one or more of silicon (Si), silicon carbide (SiC) and silicon oxide (SiO_{q}, 0.8≤q≤2.5).

Moreover, the silicon material may be included at 1 to 20 wt% with respect to the total weight of the negative electrode active material.

### [Advantageous Effects]

An electrolyte additive according to the present disclosure forms a coating film on an electrode surface in the activation of a secondary battery, so the generation of a large amount of gas under a high temperature condition can be prevented, and a cell OCV drop and a decrease in capacity retention rate, which are caused by elution of metal ions from an electrode, can be effectively prevented, resulting in effective improvement in durability, performance and high-temperature safety of the battery.

### [Brief Description of the Drawings]

FIG. 1 is a graph showing the results of Raman spectroscopy of electrolyte compositions respectively prepared in Example 1 and Comparative Example 1 according to the present disclosure.
FIG. 2 is a graph showing the results of differential capacity curve analysis for half-cells including the electrolyte compositions respectively prepared in Example 1 and Comparative Example 1 according to the present disclosure.
FIG. 3 is a graph showing the results of linear sweep voltammetry for three-electrode batteries including the electrolyte compositions respectively prepared in Example 1 and Comparative Example 1 according to the present disclosure.
FIGS. 4A and 4B are a set of graphs showing the results of X-ray photoelectron spectroscopy for coating films formed on positive electrodes surfaces of each lithium secondary battery prepared in Example 13 and Comparative Examples 13 and 16 according to the present disclosure, respectively, after charging/discharging.
FIGS. 5A and 5B are a set of graphs showing the results of X-ray photoelectron spectroscopy for coating films formed on negative electrodes surfaces of each lithium secondary battery including the electrolyte composition prepared in Example 13 and Comparative Examples 13 and 16 according to the present disclosure, respectively, after charging/discharging.

### [Best Mode for Carrying Out the invention]

The present disclosure may have various modifications and various examples, and thus specific examples are illustrated in the drawings and described in detail in the detailed description.

The terms "comprise," "include" and "have" used herein designate the presence of characteristics, numbers, steps, actions, components or members described in the specification or a combination thereof, and it should be understood that the possibility of the presence or addition of one or more other characteristics, numbers, steps, actions, components, members or a combination thereof is not excluded in advance.

In addition, when a part of a layer, film, region or plate is disposed "on" another part, this includes not only a case in which one part is disposed "directly on" another part, but a case in which a third part is interposed therebetween. In contrast, when a part of a layer, film, region or plate is disposed "under" another part, this includes not only a case in which one part is disposed "directly under" another part, but a case in which a third part is interposed therebetween. In addition, in this application, "on" may include not only a case of disposed on an upper part but also a case of disposed on a lower part.

In addition, in the present disclosure, the "included as a main component" may mean that a defined component is included at 50 wt% or more, 60 wt% or more, F70 wt% or more, 80 wt% or more, 90 wt% or more, or 95 wt% or more with respect to the total weight. For example, the "graphite is included as a main ingredient in a negative electrode active material" means that graphite is included at 50 wt% or more, 60 wt% or more, 70 wt% or more, 80 wt% or more, 90 wt% or more, or 95 wt% or more with respect to the total weight of the negative electrode active material, and in some cases, it means that a negative electrode active material totally consists of graphite and thus includes 100 wt% of graphite.

Hereinafter, the present disclosure will be described in further detail.

### Electrolyte additive for secondary battery

One embodiment of the present disclosure provides
a lithium secondary battery as claimed, which comprises and electrolyte composition for a lithium secondary battery comprising a non-aqueous organic solvent;
a lithium salt; and
a compound represented by Formula 1: 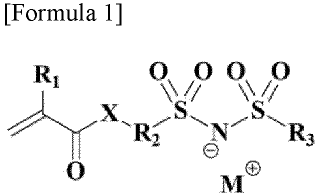
wherein,
R₁ is hydrogen or an alkyl group having 1 to 4 carbon atoms,
R₂ includes one or more selected from an alkylene group having 1 to 10 carbon atoms, an oxyalkylene group having 1 to 10 carbon atoms, a cycloalkylene group having 5 to 10 carbon atoms, and 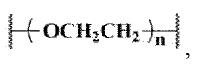
R₃ is a fluoro group, an alkyl group having 1 to 10 carbon atoms, an alkoxy group having 1 to 10 carbon atoms, or 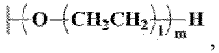
wherein one or more hydrogens included in the alkyl groups, the alkylene group, the alkoxy group, the oxyalkylene group, the cycloalkylene group, 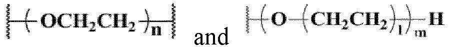 are optionally substituted with a fluorine atom,
X is an oxygen atom (O) or -NR₄, wherein R₄ is hydrogen or an alkyl group having 1 to 4 carbon atoms,
M includes one or more selected from the group consisting of lithium, sodium, potassium, tetraalkyl ammonium having 1 to 4 carbon atoms, and tetraalkyl phosphonium having 1 to 4 carbon atoms,
1 is an integer of 1 to 6, and
each of m and n is an integer of 2 to 20.

The electrolyte additive for a secondary battery according to the present disclosure includes an ionic compound including a mother nucleus to which a (meth)acrylate group or an acrylamide group is bonded via a functional group that includes a saturated hydrocarbon chain or a structure to which an oxygen atom is introduced to a saturated hydrocarbon chain, at one side based on a sulfonylimide group as shown in Formula 1. As the compound has a structure including a (meth)acrylate group or an acrylamide group bonded at one side based on a sulfonylimide group using a functional group that has an alkylene structure of a saturated hydrocarbon chain or an ether group formed by introducing an oxygen atom to a saturated hydrocarbon chain as a linker, and having a charge in this molecule, an organic and/or inorganic coating film may be uniformly formed on the surface of the positive electrode and/or the negative electrode in the activation of the secondary battery including the electrolyte additive. Therefore, the electrolyte addictive may be directly included in the organic and/or inorganic coating film formed on the surface of the positive electrode and/or the negative electrode in the form of a monomolecular(or monomer) in the activation of a secondary battery, and thus, the electrolyte additive can inhibit gas generation due to decomposition of the electrolyte when the battery is exposed to a high temperature, and improve the OCV drop phenomenon and capacity reduction of the battery, which occur at the positive electrode.

To this end, in the compound represented by Formula 1,
R₁ is hydrogen, a methyl group, an ethyl group or a propyl group,
R₂ includes one or more selected from a methylene group, an ethylene group, a propylene group, a butylene group, an oxymethylene group, an oxyethylene group, an oxypropylene group, an oxybutylene group, a cyclobutylene group, a cyclopentylene group, a cyclohexylene group, a cycloheptylene group, 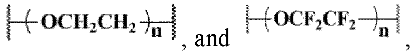
R₃ is a fluoro group, a methyl group, an ethyl group, a propyl group, a butyl group, a methoxy group, an ethoxy group, a propoxy group, 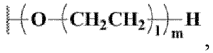 or 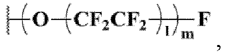
X is an oxygen atom (O), -NH, -NCH₃, or -NCH₂CH₃,
M includes one or more selected from the group consisting of lithium, sodium, tetramethyl ammonium having 1 to 4 carbon atoms, and tetraalkyl phosphonium having 1 to 4 carbon atoms, 1 is an integer of 1 to 6, and each of m and n is an integer of 2 to 20.

Specifically, R₁ may be hydrogen or a methyl group,
R₂ comprises one or more selected from a methylene group, an ethylene group, a propylene group, an oxymethylene group, an oxyethylene group, an oxypropylene group, a cyclobutylene group, a cyclopentylene group, a cyclohexylene group, a cycloheptylene group, 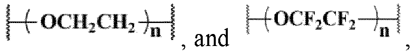
R₃ may be a fluoro group, a methyl group, an ethyl group, a propyl group, a methoxy group, an ethoxy group, 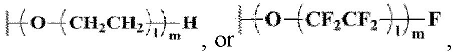
X may be an oxygen atom (O), -NH, or -NCH₃,
M may be lithium, 1 may be an integer of 1 or 2, and each of m and n may be an integer of 2 to 10.

In one example, the compound represented by Formula 1 may be one or more compounds selected from Structural Formula 1 to Structural Formula 120 below:

| | | |
|---|---|---|
| Structural Formula 1 | Structural Formula 2 | Structural Formula 3 |
| 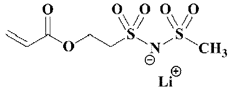 | 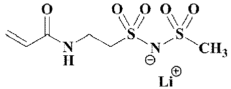 | 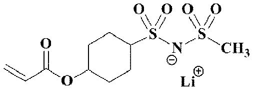 |
| Structural Formula 4 | Structural Formula 5 | Structural Formula 6 |
| 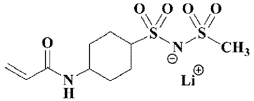 | 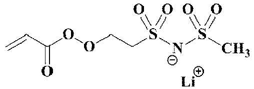 | 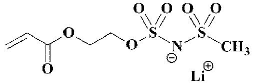 |
| Structural Formula 7 | Structural Formula 8 | Structural Formula 9 |
| 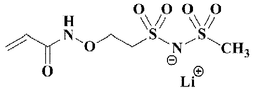 | 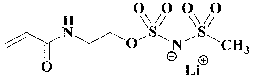 | 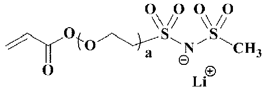 |
| Structural Formula 10 | Structural Formula 11 | Structural Formula 12 |
| 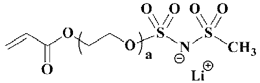 | 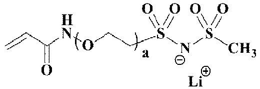 | 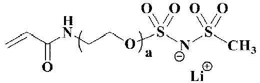 |

| | | |
|---|---|---|
| | Structural Formula 14 | Structural Formula 15 |
| | 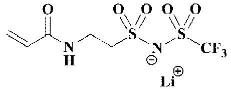 | 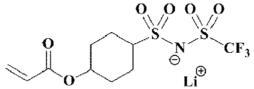 |
| Structural Formula 16 | Structural Formula 17 | Structural Formula 18 |
| 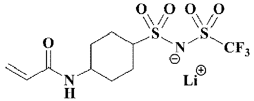 | 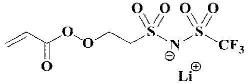 | 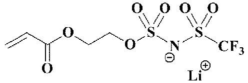 |
| Structural Formula 19 | Structural Formula 20 | Structural Formula 21 |
| 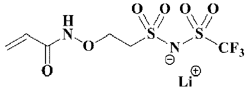 | 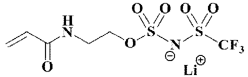 | 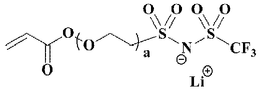 |
| Structural Formula 22 | Structural Formula 23 | Structural Formula 24 |
| 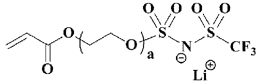 | 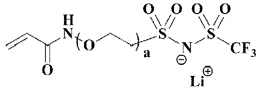 | 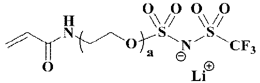 |

| | | |
|---|---|---|
| Structural Formula 25 | Structural Formula 26 | Structural Formula 27 |
| 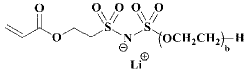 | 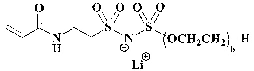 | 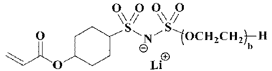 |
| Structural Formula 28 | Structural Formula 29 | Structural Formula 30 |
| 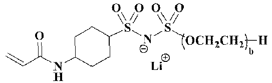 | 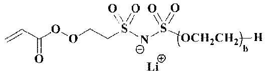 | 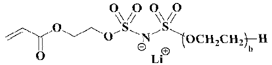 |
| Structural Formula 31 | Structural Formula 32 | Structural Formula 33 |
| 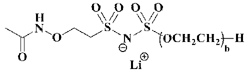 | 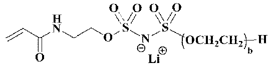 | 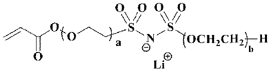 |
| Structural Formula 34 | Structural Formula 35 | Structural Formula 36 |
| 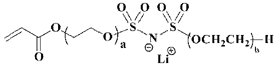 | 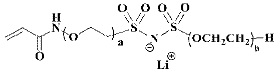 | 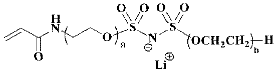 |

| | | |
|---|---|---|
| Structural Formula 37 | Structural Formula 38 | Structural Formula 39 |
| 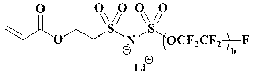 | 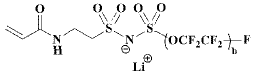 | 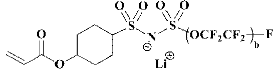 |
| Structural Formula 40 | Structural Formula 41 | Structural Formula 42 |
| 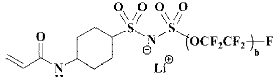 | 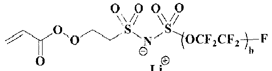 | 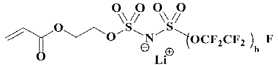 |
| Structural Formula 43 | Structural Formula 44 | Structural Formula 45 |
| 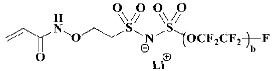 | 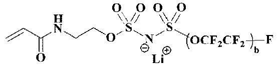 | 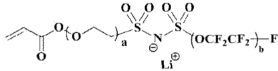 |
| Structural Formula 46 | Structural Formula 47 | Structural Formula 48 |
| 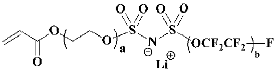 | 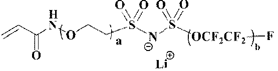 | 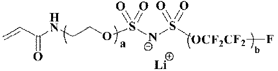 |

| | | |
|---|---|---|
| Structural Formula 49 | Structural Formula 50 | Structural Formula 51 |
| 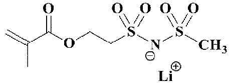 | 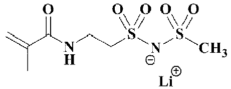 | 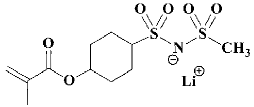 |
| Structural Formula 52 | Structural Formula 53 | Structural Formula 54 |
| 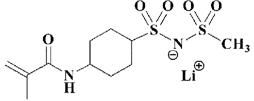 | 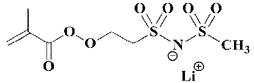 | 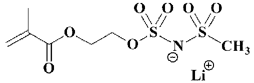 |
| Structural Formula 55 | Structural Formula 56 | Structural Formula 57 |
| 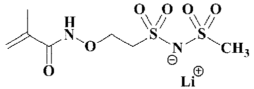 | 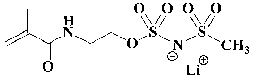 | 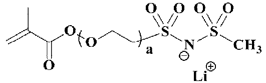 |
| Structural Formula 58 | Structural Formula 59 | Structural Formula 60 |
| 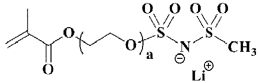 | 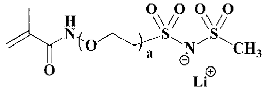 | 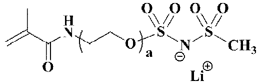 |

| | | |
|---|---|---|
| | Structural Formula 62 | Structural Formula 63 |
| | 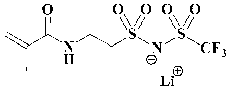 | 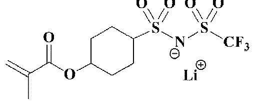 |
| Structural Formula 64 | Structural Formula 65 | Structural Formula 66 |
| 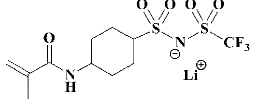 | 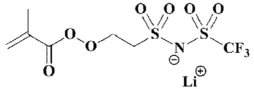 | 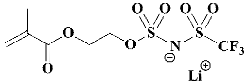 |
| Structural Formula 67 | Structural Formula 68 | Structural Formula 69 |
| 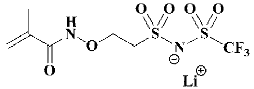 | 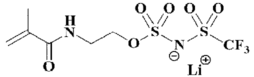 | 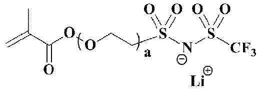 |
| Structural Formula 70 | Structural Formula 71 | Structural Formula 72 |
| 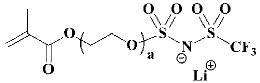 | 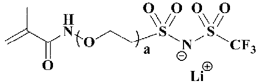 | 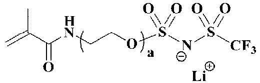 |

| | | |
|---|---|---|
| Structural Formula | Structural Formula 74 | Structural Formula |
| 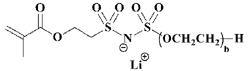 | 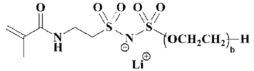 | 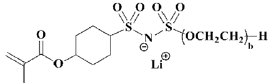 |
| Structural Formula 76 | Structural Formula 77 | Structural Formula 78 |
| 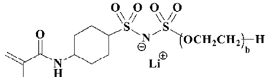 | 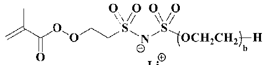 | 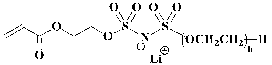 |
| Structural Formula 79 | Structural Formula 80 | Structural Formula 81 |
| 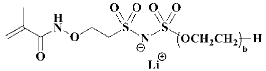 | 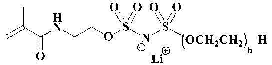 | 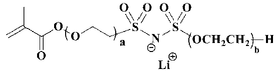 |
| Structural Formula 82 | Structural Formula 83 | Structural Formula 84 |
| 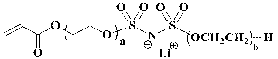 | 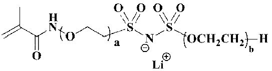 | 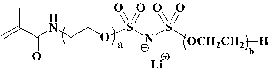 |

| | | |
|---|---|---|
| Structural Formula 85 | Structural Formula 86 | Structural Formula 87 |
| 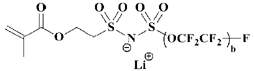 | 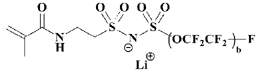 | 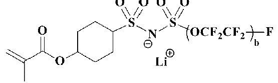 |
| Structural Formula 88 | Structural Formula 89 | Structural Formula 90 |
| 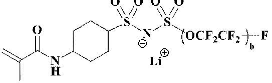 | 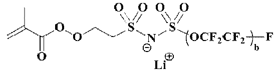 | 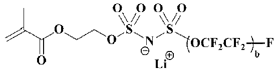 |
| Structural Formula 91 | Structural Formula 92 | Structural Formula 93 |
| 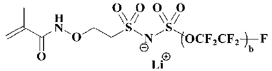 | 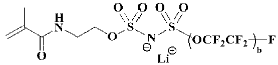 | 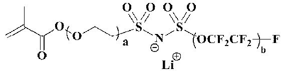 |
| Structural Formula 94 | Structural Formula 95 | Structural Formula 96 |
| 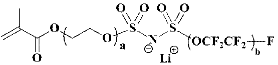 | 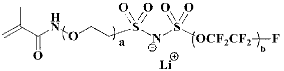 | 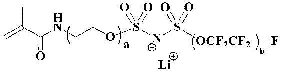 |

| | | |
|---|---|---|
| Structural Formula 97 | Structural Formula 98 | Structural Formula 99 |
| 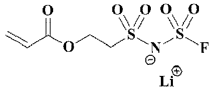 | 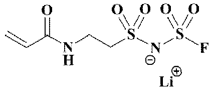 | 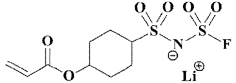 |
| Structural Formula 100 | Structural Formula 101 | Structural Formula 102 |
| 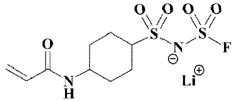 | 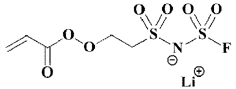 | 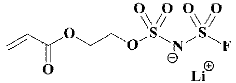 |
| Structural Formula 103 | Structural Formula 104 | Structural Formula 105 |
| 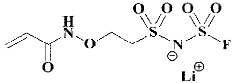 | 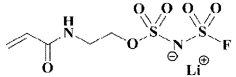 | 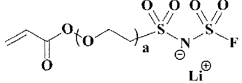 |
| Structural Formula 106 | Structural Formula 107 | Structural Formula 108 |
| 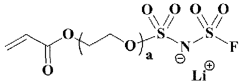 | 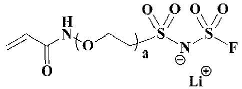 | 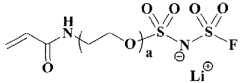 |

| | | |
|---|---|---|
| Structural Formula 109 | Structural Formula 110 | Structural Formula 111 |
| 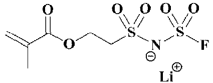 | 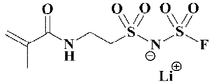 | 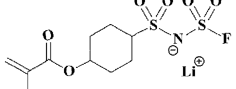 |
| Structural Formula 112 | Structural Formula 113 | Structural Formula 114 |
| 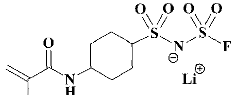 | 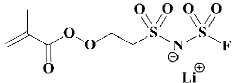 | 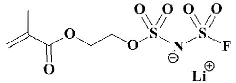 |
| Structural Formula 115 | Structural Formula 116 | Structural Formula 117 |
| 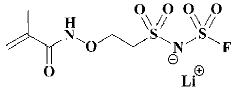 | 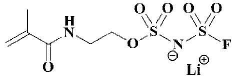 | 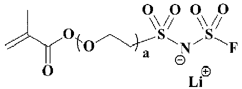 |
| Structural Formula 118 | Structural Formula 119 | Structural Formula 120 |
| 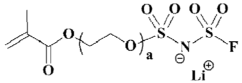 | 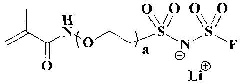 | 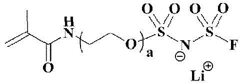 |

In Structural Formula 9~12, 21~48, 57~60, 69~96, 105~108, and 117~120 a and b is an integer of 2 to 4 respectively.

Since the electrolyte additive according to the present disclosure, as described above, has a structure that has a (meth)acrylate group or an acrylamide group binding via a functional group that has an alkylene structure of a saturated hydrocarbon chain or an ether group formed by introducing an oxygen atom to a saturated hydrocarbon chain and has a charge in the molecule, at one side based on a sulfonylimide group, the electrolyte additive may be directly involved in a solvation shell of lithium ions even under a low potential in the activation of the battery to uniformly form an inorganic coating film through a reduction reaction on the surface of the negative electrode and to uniformly form an inorganic coating film through an oxidation reaction on the surface of the positive electrode at the same time.

Therefore, the electrolyte additive can inhibit gas generation caused by decomposition of the electrolyte when the battery is exposed to a high temperature, and improve the OCV drop phenomenon and capacity reduction of the battery, which occur at the positive electrode, resulting in the prevention of the deterioration of a secondary battery and further improvement in high-temperature safety.

### Electrolyte composition for lithium secondary battery

In addition, one embodiment of the present disclosure provides
an electrolyte composition for a lithium secondary battery, which includes a non-aqueous organic solvent; a lithium salt; and a compound represented by Formula 1, wherein the compound represented by Formula 1 is one or more compounds selected from Structural Formula 1 to Structural Formula 120 below :

| | | |
|---|---|---|
| Structural Formula 1 | Structural Formula 2 | Structural Formula 3 |
| 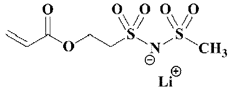 | 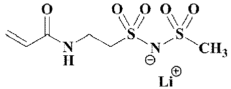 | 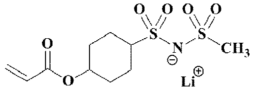 |
| Structural Formula 4 | Structural Formula 5 | Structural Formula 6 |
| 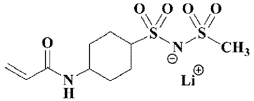 | 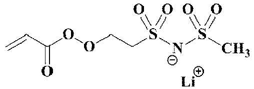 | 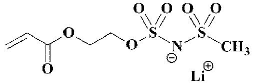 |
| Structural Formula 7 | Structural Formula 8 | Structural Formula 9 |
| 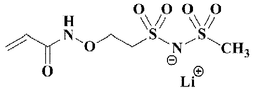 | 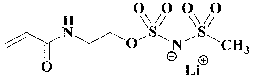 | 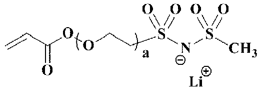 |
| Structural Formula 10 | Structural Formula 11 | Structural Formula 12 |
| 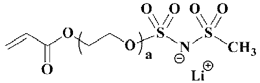 | 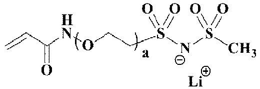 | 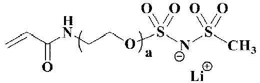 |

| | | |
|---|---|---|
| | Structural Formula 14 | Structural Formula 15 |
| | 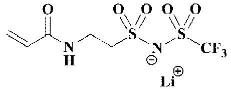 | 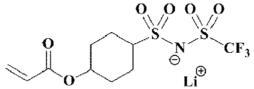 |
| Structural Formula 16 | Structural Formula 17 | Structural Formula 18 |
| 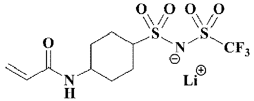 | 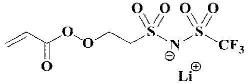 | 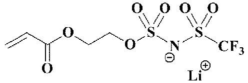 |
| Structural Formula 19 | Structural Formula 20 | Structural Formula 21 |
| 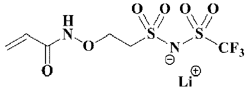 | 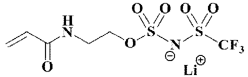 | 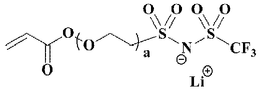 |
| Structural Formula 22 | Structural Formula 23 | Structural Formula 24 |
| 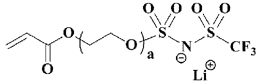 | 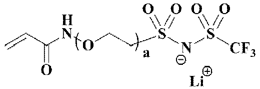 | 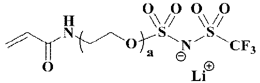 |

| | | |
|---|---|---|
| Structural Formula 25 | Structural Formula 26 | Structural Formula 27 |
| 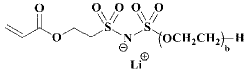 | 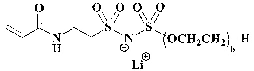 | 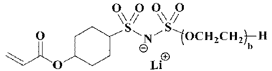 |
| Structural Formula 28 | Structural Formula 29 | Structural Formula 30 |
| 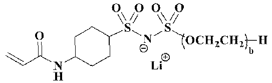 | 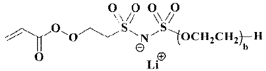 | 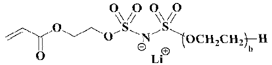 |
| Structural Formula 31 | Structural Formula 32 | Structural Formula 33 |
| 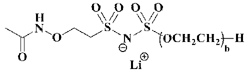 | 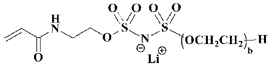 | 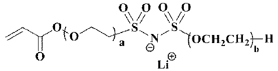 |
| Structural Formula 34 | Structural Formula 35 | Structural Formula 36 |
| 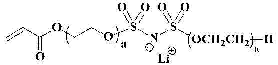 | 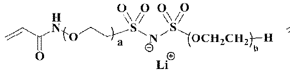 | 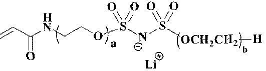 |

| | | |
|---|---|---|
| Structural Formula 37 | Structural Formula 38 | Structural Formula 39 |
| 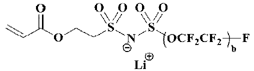 | 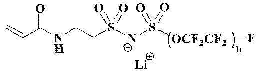 | 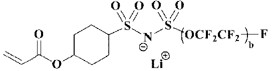 |
| Structural Formula 40 | Structural Formula 41 | Structural Formula 42 |
| 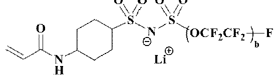 | 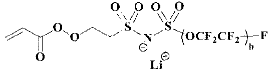 | 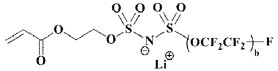 |
| Structural Formula 43 | Structural Formula 44 | Structural Formula 45 |
| 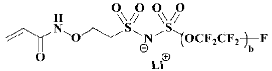 | 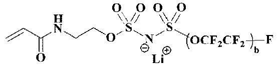 | 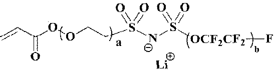 |
| Structural Formula 46 | Structural Formula 47 | Structural Formula 48 |
| 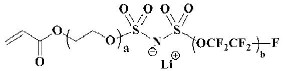 | 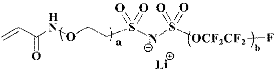 | 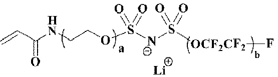 |

| | | |
|---|---|---|
| Structural Formula 49 | Structural Formula 50 | Structural Formula 51 |
| 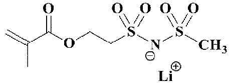 | 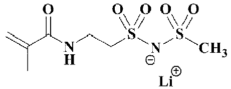 | 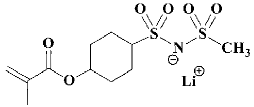 |
| Structural Formula 52 | Structural Formula 53 | Structural Formula 54 |
| 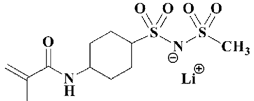 | 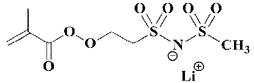 | 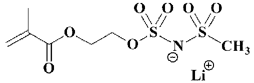 |
| Structural Formula 55 | Structural Formula 56 | Structural Formula 57 |
| 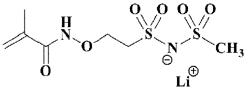 | 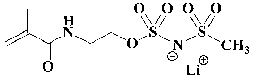 | 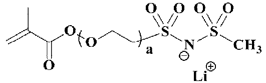 |
| Structural Formula 58 | Structural Formula 59 | Structural Formula 60 |
| 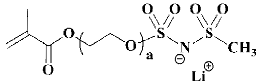 | 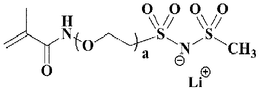 | 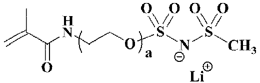 |

| | | |
|---|---|---|
| | Structural Formula 62 | Structural Formula 63 |
| | 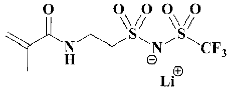 | 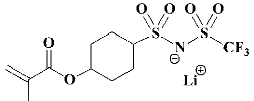 |
| Structural Formula 64 | Structural Formula 65 | Structural Formula 66 |
| 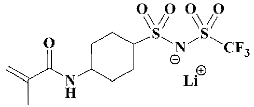 | 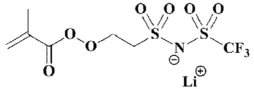 | 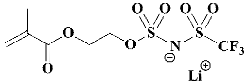 |
| Structural Formula 67 | Structural Formula 68 | Structural Formula 69 |
| 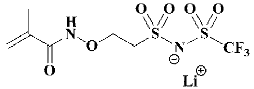 | 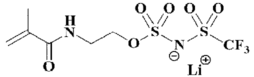 | 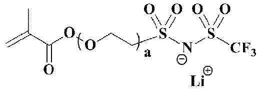 |
| Structural Formula 70 | Structural Formula 71 | Structural Formula 72 |
| 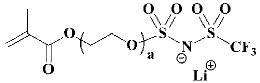 | 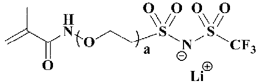 | 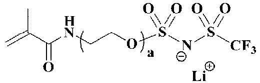 |

| | | |
|---|---|---|
| Structural Formula 73 | Structural Formula 74 | Structural Formula 75 |
| 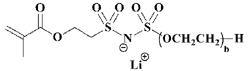 | 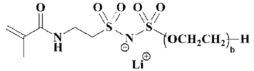 | 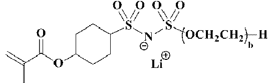 |
| Structural Formula 76 | Structural Formula 77 | Structural Formula 78 |
| 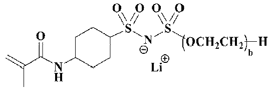 | 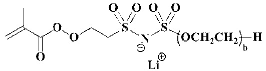 | 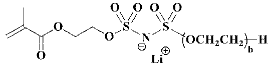 |
| Structural Formula 79 | Structural Formula 80 | Structural Formula 81 |
| 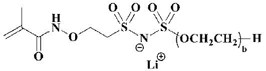 | 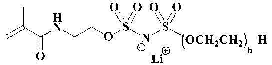 | 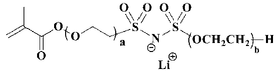 |
| Structural Formula 82 | Structural Formula 83 | Structural Formula 84 |
| 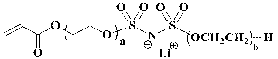 | 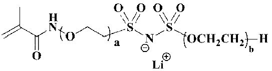 | 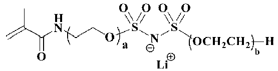 |

| | | |
|---|---|---|
| Structural Formula 85 | Structural Formula 86 | Structural Formula 87 |
| 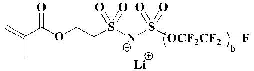 | 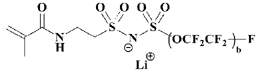 | 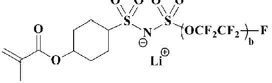 |
| Structural Formula 88 | Structural Formula 89 | Structural Formula 90 |
| 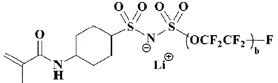 | 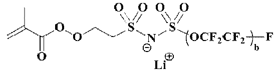 | 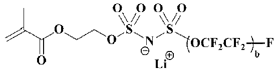 |
| Structural Formula 91 | Structural Formula 92 | Structural Formula 93 |
| 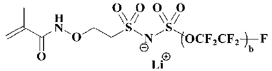 | 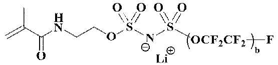 | 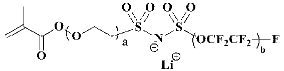 |
| Structural Formula 94 | Structural Formula 95 | Structural Formula 96 |
| 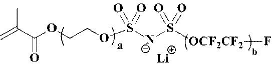 | 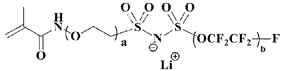 | 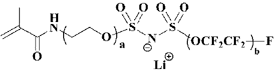 |

| | | |
|---|---|---|
| Structural Formula 97 | Structural Formula 98 | Structural Formula 99 |
| 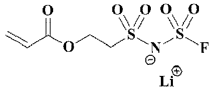 | 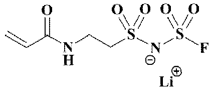 | 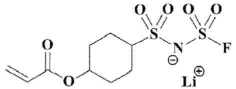 |
| Structural Formula 100 | Structural Formula 101 | Structural Formula 102 |
| 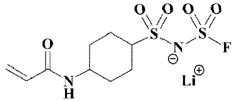 | 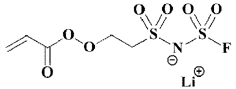 | 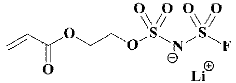 |
| Structural Formula 103 | Structural Formula 104 | Structural Formula 105 |
| 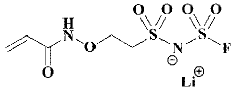 | 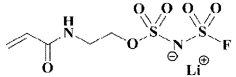 | 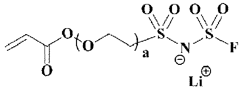 |
| Structural Formula 106 | Structural Formula 107 | Structural Formula 108 |
| 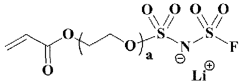 | 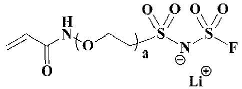 | 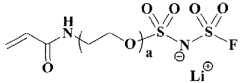 |

| | | |
|---|---|---|
| Structural Formula 109 | Structural Formula 110 | Structural Formula 111 |
| 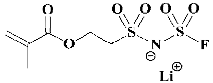 | 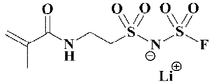 | 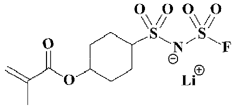 |
| Structural Formula 112 | Structural Formula 113 | Structural Formula 114 |
| 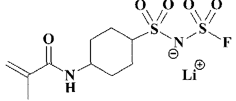 | 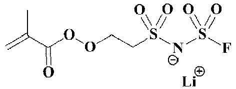 | 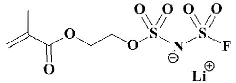 |
| Structural Formula 115 | Structural Formula 116 | Structural Formula 117 |
| 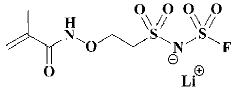 | 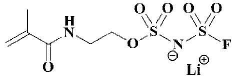 | 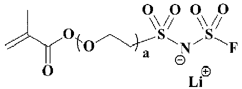 |
| Structural Formula 118 | Structural Formula 119 | Structural Formula 120 |
| 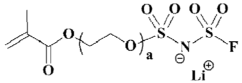 | 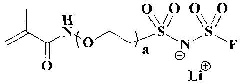 | 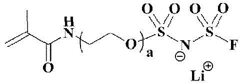 |

In Structural Formula 9~12, 21~48, 57~60, 69~96, 105~108, and 117~120 a and b is an integer of 2 to 4 respectively.

As the electrolyte composition for a lithium secondary battery according to the present disclosure is a non-aqueous electrolyte composition, it may be a liquid electrolyte which has a composition in which a lithium salt and an electrolyte additive are included in a non-aqueous organic solvent. Here, the electrolyte composition includes the above-described electrolyte additive, which includes the compound represented by Formula 1, so it may be directly involved in a solvation shell of lithium ions in the activation of the battery including the electrolyte composition, thereby uniformly forming a negatively charged and/or inorganic coating film on the surface of a negative electrode through a reduction reaction.

Here, the compound represented by Formula 1 may be included at a certain content in the electrolyte composition. Specifically, the compound represented by Formula 1 may be included at 0.01 to 5 wt%, and more specifically, 0.05 to 3 wt% or 1.0 to 2.5 wt% with respect to the total weight of the electrolyte composition. In the present disclosure, the degradation of wettability of an electrode and a separator due to the increase the viscosity of the electrolyte composition, when the content of the electrolyte additive is used in excess outside the above range, is prevented. Furthermore, a polymerization by self-reaction is induced in the electrolyte composition, thereby preventing degraded battery performance due to a reduction in ion conductivity of the electrolyte composition. In addition, the present disclosure may prevent the additive effect from being insignificantly implemented when the electrolyte additive is used at a trace amount outside the above range.

Meanwhile, the lithium salt used in the electrolyte composition may be applied without particular limitation as long as it is used in a non-aqueous electrolyte in the art. Specifically, the lithium salt may include one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

The concentration of the lithium salt is not particularly limited, and the lower limit of the appropriate concentration range is 0.5 mol/L or more, specifically 0.7 mol/L or more, and more specifically 0.9 mol/L or more, and the upper limit of the appropriate concentration range is 2.5 mol/L or less, specifically 2.0 mol/L or less, and more specifically 1.5 mol/L or less. When the concentration of the lithium salt is lower than 0.5 mol/L, there is a risk that ion conductivity is reduced, and the cycle characteristics and output characteristics of a non-aqueous electrolyte battery are lowered. In addition, when the concentration of the lithium salt exceeds 2.5 mol/L, the viscosity of an electrolyte for non-aqueous electrolyte battery increases, and thus there is a risk of decreasing ion conductivity and lowering the cycle characteristics and output characteristics of a non-aqueous electrolyte battery.

In addition, when a large amount of lithium salt is dissolved in a non-aqueous organic solvent at one time, the liquid temperature may increase because of the dissolution heat for the lithium salt. As described above, when the temperature of the non-aqueous organic solvent significantly increases due to the dissolution heat for the lithium salt, there is a risk that the decomposition may be accelerated to generate hydrogen fluoride (HF). Hydrogen fluoride (HF) is not preferable because it causes degraded battery performance. Therefore, a temperature at which the lithium salt is dissolved in a non-aqueous organic solvent may be adjusted to -20 to 80 °C, and specifically 0 to 60 °C, but the present disclosure is not particularly limited thereto.

Furthermore, a non-aqueous organic solvent used in the electrolyte composition may be applied without particular limitation as long as it can be used in a non-aqueous electrolyte in the art. Specifically, examples of the non-aqueous organic solvents may include aprotic organic solvents such as N-methyl-2-pyrrolidinone, ethylene carbonate (EC), propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, a dioxolane derivative, sulfolane, methyl sulfolane, 1,3-diethyl-2-imidazolidinone, a propylene carbonate derivative, a tetrahydrofuran derivative, ether, methyl propionate, and ethyl propionate.

In addition, as a non-aqueous solvent used in the present disclosure, one type of the above examples may be used alone, or two or more types thereof may be used by mixing in any combination and ratio according to purpose. In terms of electrochemical stability against the oxidation/reduction of the solvent and chemical stability against heat or the reaction with a solute, among the above examples, particularly, propylene carbonate, ethylene carbonate, fluoroethylene carbonate, diethyl carbonate, dimethyl carbonate, or ethyl methyl carbonate is preferable.

Meanwhile, the electrolyte composition may further include an additive, other than the above-described basic components. Without departing the gist of the present disclosure, an additive generally used in the non-aqueous electrolyte of the present disclosure may be added at any ratio. Specifically, the additive may be a compound having an overcharge prevention effect, a negative electrode coating film-forming effect, and a positive electrode protection effect, such as cyclohexylbenzene, biphenyl, t-butylbenzene, vinylene carbonate, vinylethylene carbonate, difluoroanisole, fluoroethylene carbonate, propane sultone, succinonitrile, or dimethylvinylene carbonate. In addition, in the case of use in a non-aqueous electrolyte battery called a lithium polymer battery, it is also possible to use an electrolyte for a non-aqueous electrolyte battery after being pseudo-solidified by a gelling agent or cross-linked polymer.

### Lithium secondary battery

Further, one embodiment of the present disclosure provides a lithium secondary battery, which includes:
an electrode assembly that includes a positive electrode including one or more positive electrode active materials of lithium metal oxides represented by Formulas 2 and 3 below, a negative electrode, and a separator interposed between the positive electrode and the negative electrode; and
the electrolyte composition according to the present disclosure:

   [Formula 2] Liₓ[Ni_{y}Co_{z}Mn_{w}M¹ᵥ]O₂

   [Formula 3] LiM²ₚMn₍₂₋ₚ₎O₄

In Formula 2 and Formula 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6.

The lithium secondary battery according to the present disclosure includes a positive electrode including a positive electrode active material, a negative electrode including a negative electrode active material, a separator interposed between the positive electrode and the negative electrode, and the lithium salt-containing non-aqueous electrolyte composition of the present disclosure, which is described above.

Specifically, the positive electrode includes a positive electrode mixture layer formed by applying, drying, and pressing a positive electrode active material on a positive electrode current collector, and may selectively further include a conductive material, a binder, or other additives as needed.

Here, the positive electrode active material is a material that can cause an electrochemical reaction on the positive electrode current collector and may include one or more lithium metal oxides represented by Formula 2 and Formula 3, which enable reversible intercalation and deintercalation of lithium ions.

The lithium metal oxides represented by Formula 2 and Formula 3 are materials containing nickel (Ni) and manganese (Mn) at high contents, respectively, and have advantages of stably supplying high capacity and/or high voltage electricity when used as a positive electrode active material. In addition, the activation of the secondary battery requires a high charging potential of about 4.0V or more to form a coating film on the surface of the positive electrode and/or the negative electrode. Unlike the conventional positive electrode active materials with a charging potential of less than 4.0V such as iron phosphate, the lithium metal oxides have a high charging potential of about 4.0 or more, and thus, a coating film may be formed easily on the electrode.

Here, examples of the lithium metal oxides represented by Formula 2 may include LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, and LiNi_{0.7}Co_{0.1}Mn_{0.1}Al_{0.1}O₂, and examples of the lithium metal oxides represented by Formula 3 may include LiNi_{0.7}Mn_{1.3}O₄; LiNi_{0.5}Mn_{1.5}O₄; and LiNi_{0.3}Mn_{1.7}O₄, and these oxides may be used alone or in combination.

In addition, in the positive electrode, as a positive electrode current collector, a material that does not cause a chemical change in the corresponding battery and has high conductivity may be used. For example, stainless steel, aluminum, nickel, titanium, or calcined carbon may be used and in the case of aluminum or stainless steel, one that is surface treated with carbon, nickel, titanium or silver may also be used. In addition, the average thickness of the current collector may be suitably selected within 3 to 500 µm in consideration of the conductivity and total thickness of the positive electrode to be formed.

In addition, the negative electrode, like the positive electrode, includes a negative electrode mixture layer formed by applying, drying and pressing a negative electrode active material on a negative electrode current collector, and may selectively further include a conductive material, a binder, or other additives as needed.

The negative electrode active material may include a carbon material and a silicon material. Specifically, the carbon material refers to a material that has a carbon atom as the main component, and examples of the carbon materials may include one or more selected from the group consisting of natural graphite, artificial graphite, expanded graphite, non-graphitizing carbon, carbon black, acetylene black, and Ketjen black. In addition, the silicon material refers to a material that has a silicon atom as the main component, and may include silicon (Si), silicon carbide (SiC), silicon monoxide (SiO) or silicon dioxide (SiO₂) alone or in combination. When, as the silicon (Si)-containing materials, silicon monoxide (SiO) and silicon dioxide (SiO₂) are uniformly mixed or combined to be included in the negative electrode mixture layer, these materials may be represented as a silicon oxide (SiO_{q}, 0.8≤q≤2.5).

In addition, the silicon material may be included at 1 to 20 wt%, and specifically, 3 to 10 wt%, 8 to 15 wt%, 13 to 18 wt%, or 2 to 8 wt% with respect to the total weight of the negative electrode active material. The present disclosure may maximize the energy density of the battery by controlling the content of the silicon material in the above content range.

In addition, the negative electrode current collector is not particularly limited as long as it has high conductivity without causing a chemical change in the battery, and for example, copper, stainless steel, nickel, titanium or calcined carbon may be used, and in the case of copper or stainless steel, one whose surface is treated with carbon, nickel, titanium or silver may be used. Furthermore, the average thickness of the negative electrode current collector may be suitably selected within 1 to 500 µm in consideration of the conductivity and total thickness of the negative electrode to be formed.

Meanwhile, the separator interposed between the positive electrode and the negative electrode of each unit cell is an insulating thin film having high ion permeability and high mechanical strength, and is not particularly limited as long as it is one that is commonly used in the art. Specifically, the separator may include one or more polymers selected from chemical-resistant and hydrophobic polypropylene, polyethylene and a polyethylene-propylene copolymer. The separator may have the form of a porous polymer substrate, such as a sheet or non-woven fabric including the above-described polymer, and in some cases, have the form of a composite separator in which organic or inorganic particles on the porous polymer substrate are coated with an organic binder. In addition, the separator may have an average pore diameter of 0.01 to 10 µm, and an average thickness of 5 to 300 µm.

Further, the secondary battery includes the above-described non-aqueous electrolyte composition according to the present disclosure as an electrolyte.

The electrolyte composition includes an ionic compound represented by Formula 1 below as an electrolyte additive, which has a mother nucleus to which a (meth)acrylate group or an acrylamide group is bonded via a functional group that has an alkylene structure of a saturated hydrocarbon chain or an ether group formed by introducing an oxygen atom to a saturated hydrocarbon chain, at one side based on a sulfonylimide group: 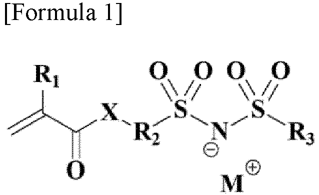 wherein,
R₁ is hydrogen or an alkyl group having 1 to 4 carbon atoms,
R₂ includes one or more selected from an alkylene group having 1 to 10 carbon atoms, an oxyalkylene group having 1 to 10 carbon atoms, a cycloalkylene group having 5 to 10 carbon atoms, and 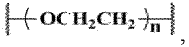
R₃ is a fluoro group, an alkyl group having 1 to 10 carbon atoms, an alkoxy group having 1 to 10 carbon atoms, or 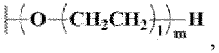
wherein one or more hydrogens included in the alkyl group, the alkylene group, the alkoxy group, the oxyalkylene group, the cycloalkylene group, 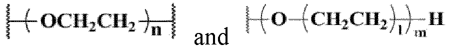 are optionally substituted with a fluorine atom,
X is an oxygen atom (O) or -NR₄, wherein R₄ is hydrogen or an alkyl group having 1 to 4 carbon atoms,
M includes one or more selected from the group consisting of lithium, sodium, potassium, tetraalkyl ammonium having 1 to 4 carbon atoms, and tetraalkyl phosphonium having 1 to 4 carbon atoms,
1 is an integer of 1 to 6, and
each of m and n is an integer of 2 to 20.

Since the compound has the structure represented by Formula 1, in the activation of a secondary battery including the same, an organic and/or inorganic coating film may be uniformly formed on the surface of a positive electrode and/or a negative electrode. To this end, the electrolyte additive may inhibit gas generation caused by the decomposition of the electrolyte when the battery is exposed to a high temperature, and improve the OCV drop phenomenon and capacity reduction of the battery, which occur at the positive electrode, resulting in further improvement in performance and high-temperature safety of the battery.

To this end, in the compound represented by Formula 1,
R₁ is hydrogen, a methyl group, an ethyl group or a propyl group,
R₂ includes one or more selected from a methylene group, an ethylene group, a propylene group, a butylene group, an oxymethylene group, an oxyethylene group, an oxypropylene group, an oxybutylene group, a cyclobutylene group, a cyclopentylene group, a cyclohexylene group, a cycloheptylene group, 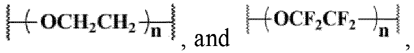
R₃ is a fluoro group, a methyl group, an ethyl group, a propyl group, a butyl group, a methoxy group, an ethoxy group, a propoxy group, 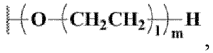 or 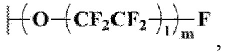
X is an oxygen atom (O), -NH, -NCH₃, or -NCH₂CH₃,
M includes one or more selected from the group consisting of lithium, sodium, tetramethyl ammonium having 1 to 4 carbon atoms, and tetraalkyl phosphonium having 1 to 4 carbon atoms, 1 is an integer of 1 to 6, and each of m and n is an integer of 2 to 20.

Specifically, R₁ may be hydrogen or a methyl group,
R₂ comprises one or more selected from a methylene group, an ethylene group, a propylene group, an oxymethylene group, an oxyethylene group, an oxypropylene group, a cyclobutylene group, a cyclopentylene group, a cyclohexylene group, a cycloheptylene group, 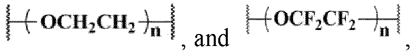
R₃ may be a fluoro group, a methyl group, an ethyl group, a propyl group, a methoxy group, an ethoxy group, 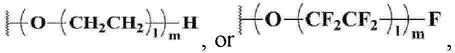
X may be an oxygen atom (O), -NH, or -NCH₃,
M may be lithium, 1 may be an integer of 1 or 2, and each of m and n may be an integer of 2 to 10.

In one example, the compound represented by Formula 1 may be any one or more compounds selected from Structural Formula 1 to Structural Formula 120:

| | | |
|---|---|---|
| Structural Formula 1 | Structural Formula 2 | Structural Formula 3 |
| 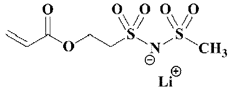 | 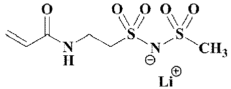 | 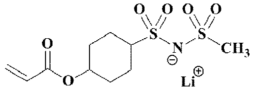 |
| Structural Formula 4 | Structural Formula 5 | Structural Formula 6 |
| 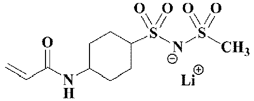 | 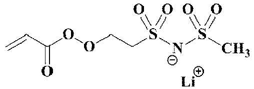 | 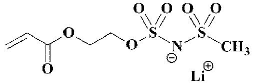 |
| Structural Formula 7 | Structural Formula 8 | Structural Formula 9 |
| 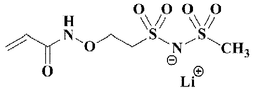 | 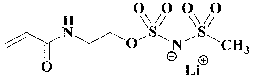 | 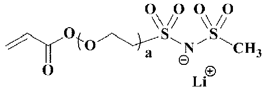 |
| Structural Formula 10 | Structural Formula 11 | Structural Formula 12 |
| 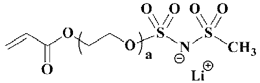 | 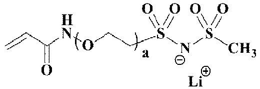 | 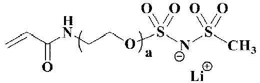 |

| | | |
|---|---|---|
| | Structural Formula 14 | Structural Formula 15 |
| | 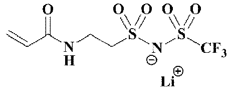 | 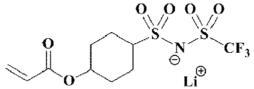 |
| Structural Formula 16 | Structural Formula 17 | Structural Formula 18 |
| 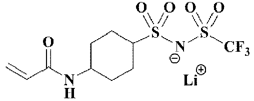 | 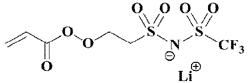 | 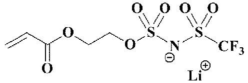 |
| Structural Formula 19 | Structural Formula 20 | Structural Formula 21 |
| 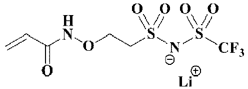 | 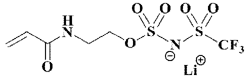 | 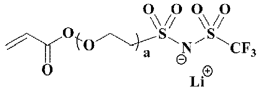 |
| Structural Formula 22 | Structural Formula 23 | Structural Formula 24 |
| 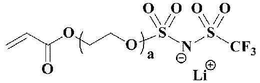 | 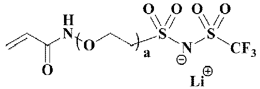 | 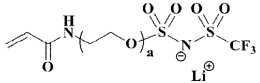 |

| | | |
|---|---|---|
| Structural Formula 25 | Structural Formula 26 | Structural Formula 27 |
| 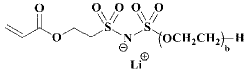 | 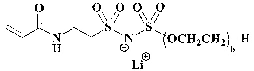 | 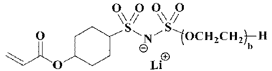 |
| Structural Formula 28 | Structural Formula 29 | Structural Formula 30 |
| 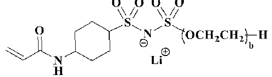 | 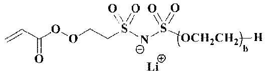 | 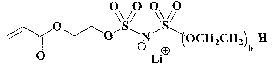 |
| Structural Formula 31 | Structural Formula 32 | Structural Formula 33 |
| 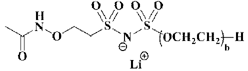 | 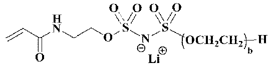 | 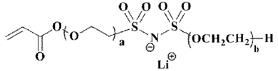 |
| Structural Formula 34 | Structural Formula 35 | Structural Formula 36 |
| 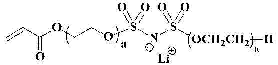 | 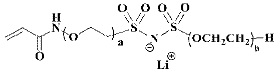 | 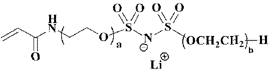 |

| | | |
|---|---|---|
| Structural Formula 37 | Structural Formula 38 | Structural Formula 39 |
| 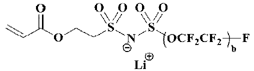 | 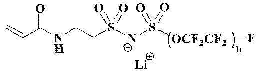 | 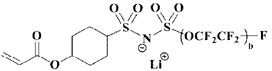 |
| Structural Formula 40 | Structural Formula 41 | Structural Formula 42 |
| 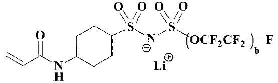 | 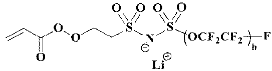 | 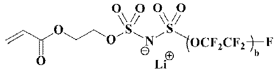 |
| Structural Formula 43 | Structural Formula 44 | Structural Formula 45 |
| 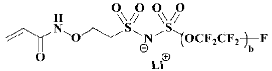 | 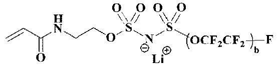 | 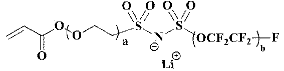 |
| Structural Formula 46 | Structural Formula 47 | Structural Formula 48 |
| 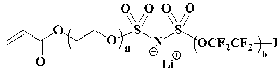 | 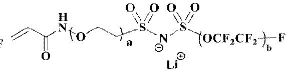 | 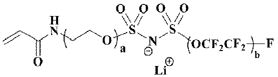 |

| | | |
|---|---|---|
| Structural Formula 49 | Structural Formula 50 | Structural Formula 51 |
| 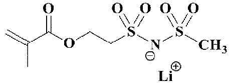 | 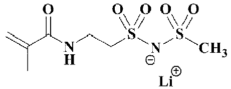 | 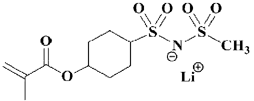 |
| Structural Formula 52 | Structural Formula 53 | Structural Formula 54 |
| 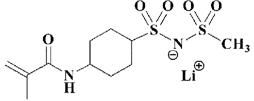 | 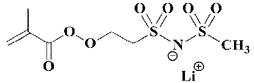 | 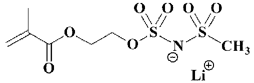 |
| Structural Formula 55 | Structural Formula 56 | Structural Formula 57 |
| 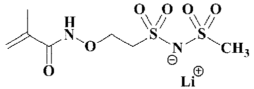 | 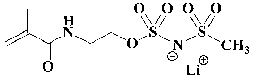 | 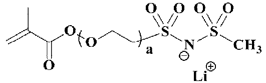 |
| Structural Formula 58 | Structural Formula 59 | Structural Formula 60 |
| 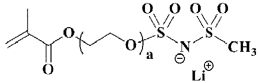 | 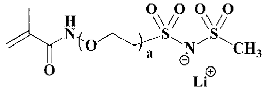 | 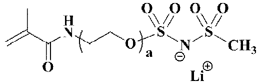 |

| | | |
|---|---|---|
| | Structural Formula 62 | Structural Formula 63 |
| | 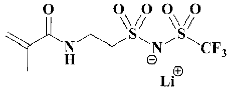 | 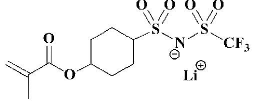 |
| Structural Formula 64 | Structural Formula 65 | Structural Formula 66 |
| 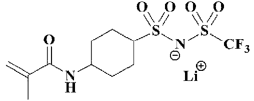 | 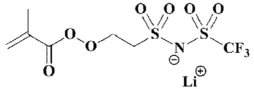 | 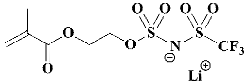 |
| Structural Formula 67 | Structural Formula 68 | Structural Formula 69 |
| 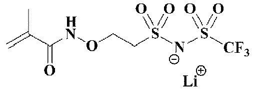 | 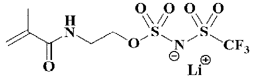 | 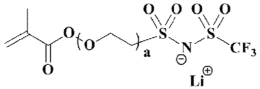 |
| Structural Formula 70 | Structural Formula 71 | Structural Formula 72 |
| 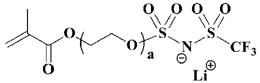 | 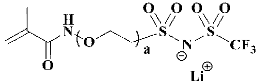 | 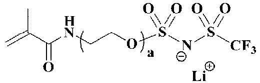 |

| | | |
|---|---|---|
| Structural Formula 73 | Structural Formula 74 | Structural Formula 75 |
| 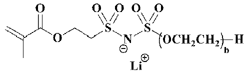 | 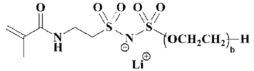 | 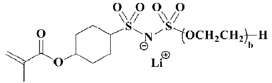 |
| Structural Formula 76 | Structural Formula 77 | Structural Formula 78 |
| 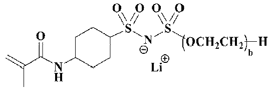 | 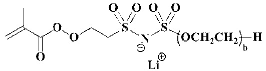 | 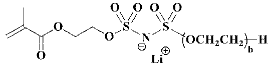 |
| Structural Formula 79 | Structural Formula 80 | Structural Formula 81 |
| 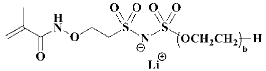 | 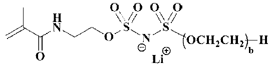 | 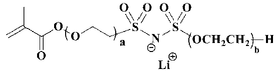 |
| Structural Formula 82 | Structural Formula 83 | Structural Formula 84 |
| 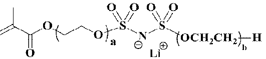 | 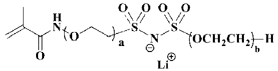 | 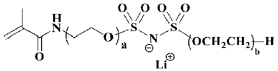 |

| | | |
|---|---|---|
| Structural Formula 85 | Structural Formula 86 | Structural Formula 87 |
| 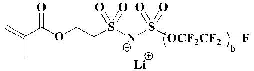 | 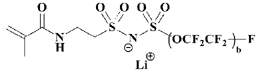 | 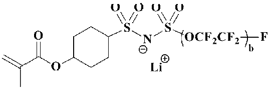 |
| Structural Formula 88 | Structural Formula 89 | Structural Formula 90 |
| 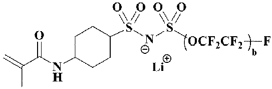 | 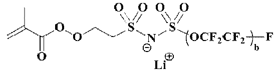 | 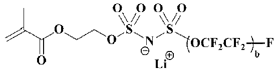 |
| Structural Formula 91 | Structural Formula 92 | Structural Formula 93 |
| 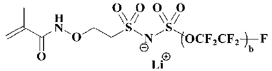 | 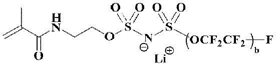 | 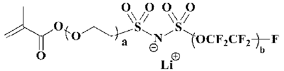 |
| Structural Formula 94 | Structural Formula 95 | Structural Formula 96 |
| 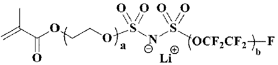 | 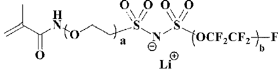 | 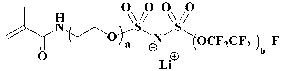 |

| | | |
|---|---|---|
| Structural Formula 97 | Structural Formula 98 | Structural Formula 99 |
| 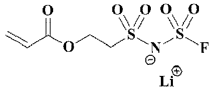 | 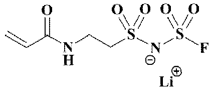 | 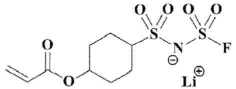 |
| Structural Formula 100 | Structural Formula 101 | Structural Formula 102 |
| 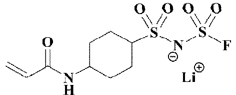 | 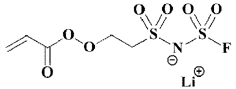 | 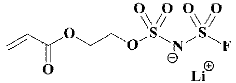 |
| Structural Formula 103 | Structural Formula 104 | Structural Formula 105 |
| 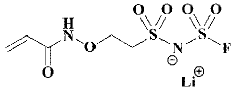 | 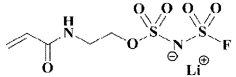 | 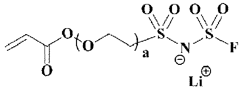 |
| Structural Formula 106 | Structural Formula 107 | Structural Formula 108 |
| 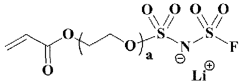 | 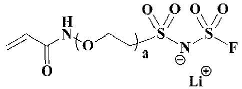 | 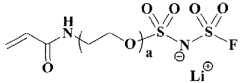 |

| | | |
|---|---|---|
| Structural Formula 109 | Structural Formula 110 | Structural Formula 111 |
| 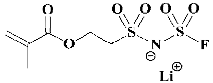 | 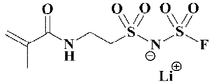 | 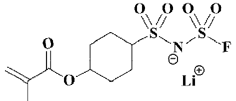 |
| Structural Formula 112 | Structural Formula 113 | Structural Formula 114 |
| 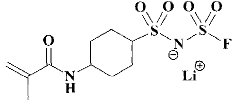 | 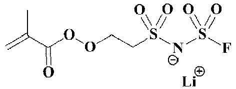 | 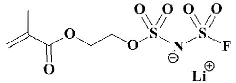 |
| Structural Formula 115 | Structural Formula 116 | Structural Formula 117 |
| 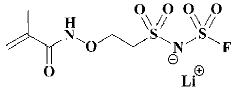 | 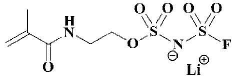 | 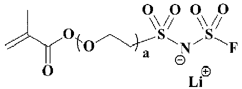 |
| Structural Formula 118 | Structural Formula 119 | Structural Formula 120 |
| 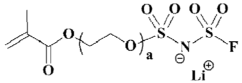 | 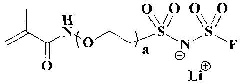 | 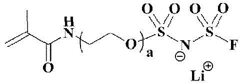 |

In Structural Formula 9~12, 21~48, 57~60, 69~96, 105~108, and 117~120 a and b is an integer of 2 to 4 respectively.

### Best Mode

Hereinafter, the present disclosure will be described in further detail with reference to examples and experimental examples.

However, the following examples and experimental example merely illustrate the present disclosure, and the content of the present disclosure is not limited to the following examples and experimental examples.

### Examples 1 to 6 and Comparative Examples 1 to 5. Preparation of electrolyte composition for lithium secondary battery

A non-aqueous electrolyte composition was prepared by dissolving 1M LiPF₆ as a lithium salt in a solvent in which ethylene carbonate (EC) and ethyl methyl carbonate (EMC) were mixed in a volume ratio of 3:7, and dissolving an additive to be 2 wt% with respect to the total weight of the electrolyte as shown in Table 1 below.

**[Table 1]**

| | Type of non-aqueous electrolyte additive |
|---|---|
| Example 1 Reference | 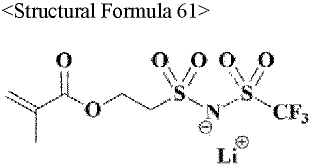 |
| Example 2 | 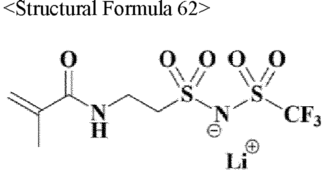 |
| Example 3 | 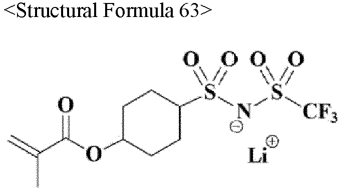 |
| Example 4 | 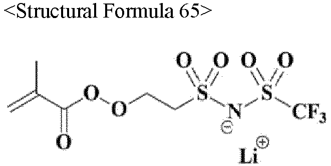 |
| Example 5 | 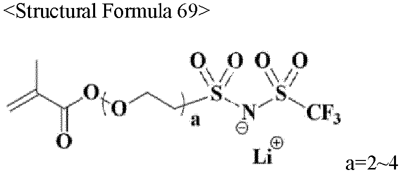 |
| Example 6 | 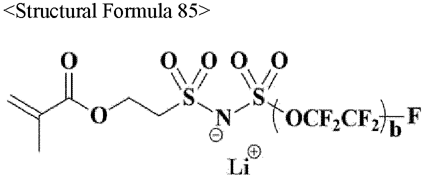 |
| Comparative Example 1 | Not added |
| Comparative Example 2 | 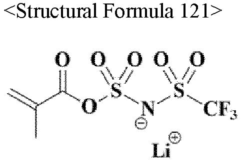 |
| Comparative Example 3 | 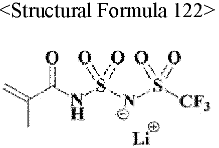 |
| Comparative Example 4 | 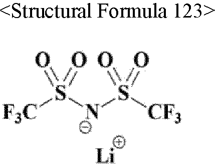 |
| Comparative Example 5 | 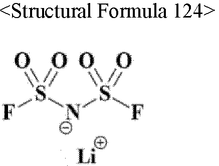 |

### Comparative example 6. Preparation of electrolyte composition for lithium secondary battery

A non-aqueous electrolyte composition was manufactured by in the same manner as in the Example 1, except that an oligomer (Weight-average molecular weight: 2,500~5,000) polymerized from the compound represented by Structural Formula 61 was used instead of the compound represented by Structural Formula 61 as the electrolyte additive.

### Examples 7 to 12 and Comparative Examples 7 to 12. Manufacture of lithium secondary battery

A positive electrode was manufactured by preparing LiNi_{0.5}Mn_{1.5}O₄ having a particle size of 5 µm as a positive electrode active material, preparing a slurry by mixing the positive electrode active material with a carbon-based conductive material and polyvinylidene fluoride as a binder in a weight ratio of 94:3:3 in N-methyl pyrrolidone (NMP), casting the slurry on an aluminum thin film, drying the slurry in a vacuum oven at 120 °C, and rolling the resultant.

Separately, a negative electrode was manufactured by preparing a negative electrode active material in which artificial graphite and silicon oxide (SiO₂) were mixed in a weight ratio of 9:1, preparing a slurry by mixing 97 parts by weight of the negative electrode active material and 3 parts by weight of styrene butadiene rubber (SBR) with water, casting the slurry on a copper thin film, drying the slurry in a vacuum oven at 130 °C, and rolling the resultant.

A lithium secondary battery was manufactured by interposing a separator consisting of 18-µm polypropylene between the positive electrode and negative electrode obtained above, inserting the resultant into a case, and injecting each of the electrolyte compositions prepared in each of Examples 1 to 6 and Comparative Examples 1 to 6 as shown in Table 2 below.

**[Table 2]**

| | Type of electrolyte composition |
|---|---|
| Example 7 | Electrolyte composition of Example 1 |
| Example 8 | Electrolyte composition of Example 2 |
| Example 9 | Electrolyte composition of Example 3 |
| Example 10 | Electrolyte composition of Example 4 |
| Example 11 | Electrolyte composition of Example 5 |
| Example 12 | Electrolyte composition of Example 6 |
| Comparative Example 7 | Electrolyte composition of Comparative Example 1 |
| Comparative Example 8 | Electrolyte composition of Comparative Example 2 |
| Comparative Example 9 | Electrolyte composition of Comparative Example 3 |
| Comparative Example 10 | Electrolyte composition of Comparative Example 4 |
| Comparative Example 11 | Electrolyte composition of Comparative Example 5 |
| Comparative Example 12 | Electrolyte composition of Comparative Example 6 |

### Experimental Example 1.

To analyze the form in which the electrolyte composition for a lithium secondary battery is present in a secondary battery, the following experiment was performed on the electrolyte compositions prepared in Example 1 and Comparative Example 1, respectively.

### Raman spectroscopy

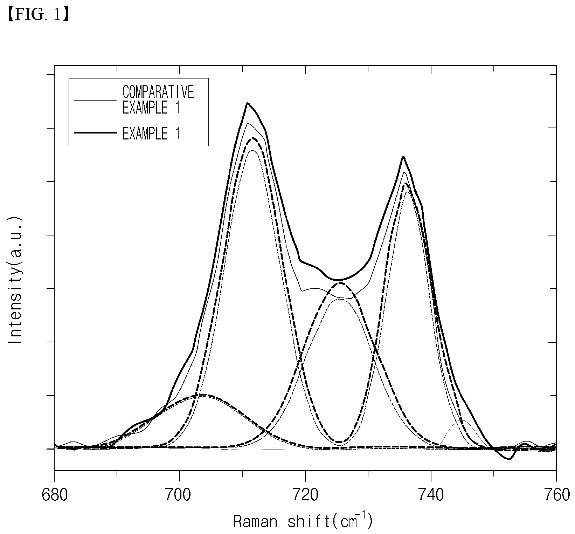

Raman spectroscopy was performed on each electrolyte composition (5 ml) using 532-mm laser at a wavelength range of 650 to 760 cm⁻¹. The result is shown in FIG. 1.

Referring to FIG. 1, it was confirmed that, compared with the electrolyte composition prepared in Comparative Example 1 not including an electrolyte additive, the electrolyte composition of Example 1 including the electrolyte additive represented by Formula 1 according to the present disclosure has an increased Raman spectrum band intensity in the vicinity of 743±1 cm⁻¹. This is a phenomenon that occurs by forming a coordinate bond between ionic materials such as a negatively charged nitrogen atom included in sulfonylimide and a positively-charged lithium ion (Li⁺) of the electrolyte additive represented by Formula 1, indicating that there is an ionic compound in the electrolyte composition of Example 1. In addition, the increase in band intensity indicates that the ionic compound can cause a reduction reaction on the surface of a negative electrode.

### Differential capacity curve analysis of half-cell

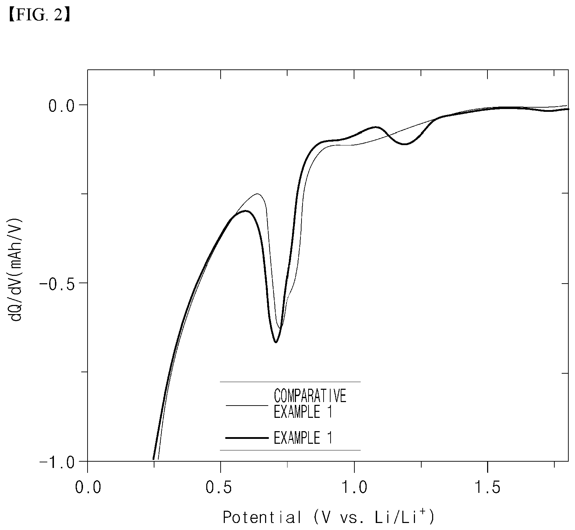

A half-cell was manufactured using lithium metal and graphite (mixture of artificial graphite:natural graphite = 9:1 (weight ratio)), and each of the electrolyte compositions prepared in Example 1 and Comparative Example 1 was injected into the half-cell. Afterward, after charging the half-cell at 25 °C from 3.5±0.5V to 0.05V at a rate of 0.005C, a potential value (V) and a capacity value (mAh) were measured, and a reduction potential value was determined by differentiating the capacitance value with respect to the potential value (dQ/dV). The result is shown in FIG. 2.

Referring to FIG. 2, it was confirmed that the electrolyte composition of Example 1 including the electrolyte additive represented by Formula 1 according to the present disclosure, unlike the electrolyte composition of Comparative Example 1 not including an electrolyte additive, shows a descending peak at a voltage near 1.32V compared to lithium. The descending peak indicates that a reduction reaction occurred on the graphite surface, which is the negative electrode, and the electrolyte additive represented by Formula 1 contained in the electrolyte composition is converted into a coating material through a reduction reaction on the surface of the negative electrode at approximately 1.32V compared to lithium.

### Evaluation of linear sweep voltammetry for three-electrode battery

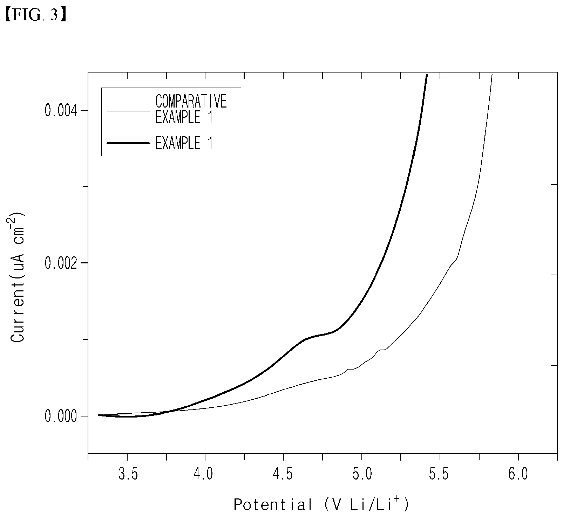

A three-electrode battery was prepared by injecting each of the electrolyte compositions prepared in Example 1 and Comparative Example 1, which includes three electrodes including two platinum electrodes and a lithium metal electrode, and linear sweep voltammetry (LSV) was performed thereon. Here, LSV was performed under the conditions of an observation range of 3.0 to 6.0V (based on lithium), a step voltage of 50 mV and a measurement rate of 50 mV/s. The result is shown in FIG. 3.

Referring to FIG. 3, it can be seen that the electrolyte composition of Example 1 including the electrolyte additive represented by Formula 1 according to the present disclosure has an increased current near 4.2V compared to lithium. This means that there is an oxidation reaction on the lithium metal surface near 4.2V, and indicates that the electrolyte additive included in the electrolyte composition of Example 1 forms a coating film through an oxidation reaction on the surface of a positive electrode under a condition of 4.2V or more, compared to lithium. In addition, it shows that the oxidation reaction is induced at a lower potential than the surface of the platinum electrode in a carbon electrode or a positive electrode, due to the catalytic properties of carbon or a transition metal.

From the above results, it can be seen that the electrolyte additive according to the present disclosure is an ionic material, which has an oxidation reaction and a reduction reaction at the positive electrode and the negative electrode, respectively, during charging/discharging of the electrode, thereby forming a coating film on the surface of each electrode.

### Experimental Example 2.

To analyze a coating film formed on an electrode surface in the activation of a lithium secondary battery according to the present disclosure and evaluate the high-temperature safety of the lithium secondary battery, the following experiment was performed. Here, the targeted lithium secondary batteries were the secondary batteries of Examples 13 to 18 and Comparative Examples 13 to 18 manufactured in the same manner as in Examples 7 to 12 and Comparative Examples 7 to 12, except that LiNi_{0.6}Co_{0.2}Mn_{0.2}O₂ and artificial graphite were used.

### Analysis of coating film on electrode surface

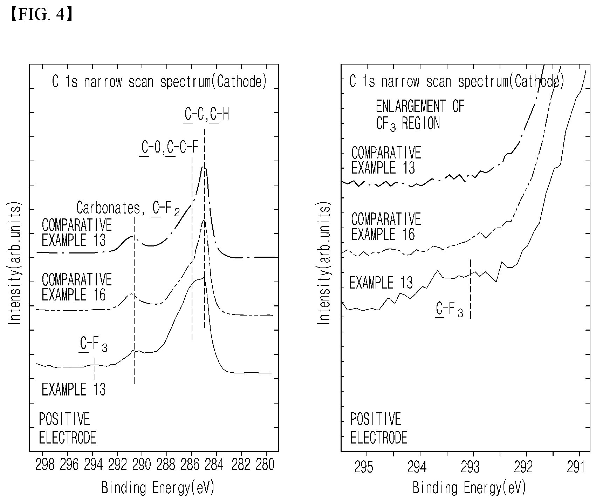

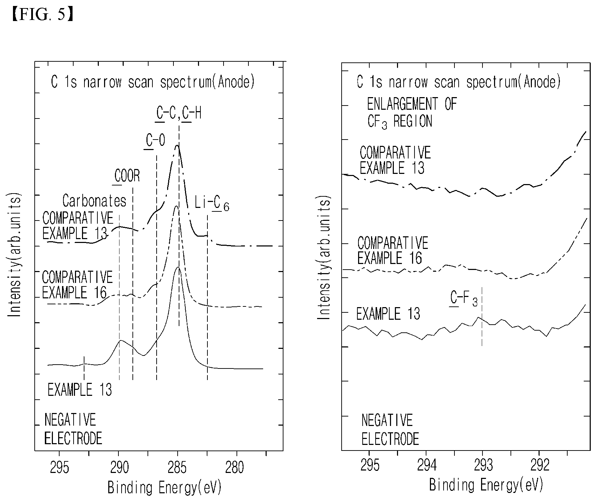

The secondary batteries of Example 13, Comparative Example 16 and Comparative Example 18 were charged/discharged three times at a charge/discharge current density of 0.33C/0.33C, a final charging voltage of 4.2V (NMC/graphite) and a final discharging voltage of 2.5V (NMC/graphite), and X-ray photoelectron spectroscopy (XPS) was performed on the surfaces of the positive and negative electrodes of each battery at full charge. The results are shown in FIGS. 4A to 4B and 5A to 5B.

Referring to FIGS. 4A-4B and 5A-5B, it showed that the secondary battery (Example 13) including the electrolyte composition of Example 1 shows peaks in the range of 280 to 300 eV indicating the carbon-fluorine binding energy at the positive and negative electrodes in X-ray photoelectron spectroscopy (XPS) performed on the electrode surface. Specifically, the positive and negative electrodes of the secondary battery showed peaks indicating the binding energy of a CF₃ group at 293±0.2 eV, which means that the electrolyte additive represented by Formula 1 contained in the electrolyte composition is involved in the formation of a coating film formed on the surfaces of the positive and negative electrodes. On the other hand, it was confirmed that the secondary battery (Comparative Example 13) using the electrolyte composition of Comparative Example 1 not including an electrolyte additive; the secondary battery (Comparative Example 16) using the electrolyte composition of Comparative Example 4 which includes the additive including sulfonylimide; the secondary battery (Comparative Example 18) using the electrolyte composition of Comparative Example 6 including the electrolyte additive in the form of oligomer do not show the binding energy peak derived from the CF₃ group at both of the positive and negative electrodes.

From the above results, it can be seen that, as the electrolyte additive according to the present disclosure includes an ionic compound in the form of monomolecular(or monomer) represented by Formula 1 including a mother nucleus to which a (meth)acrylate group or an acrylamide group is bonded via a functional group that has an alkylene structure of a saturated hydrocarbon chain or an ether group formed by introducing an oxygen atom to a saturated hydrocarbon chain, at one side based on a sulfonylimide group, a coating film which includes the ionic compound may be formed on the surface of an electrode in the activation of a secondary battery.

### Evaluation of high-temperature storage stability of secondary battery

While each secondary battery was stored at 60 °C for 56 days, ① an amount of gas generation in the secondary battery and ② OCV of the secondary battery were observed over time. And ③ capacity retention rates of each secondary battery after high-temperature storage were assessed.

Specifically, each secondary battery was charged/discharged three times at a charge/discharge current density of 0.33C/0.33C, a final charging voltage of 4.2V (NMC/graphite), and a final discharging voltage of 2.5V (NMC/graphite) to measure a battery capacity.

Afterward, each secondary battery was fully charged with 0.33C at a final charging voltage of 4.2V in a CC/CV mode, followed by starting high temperature storage. Here, the high temperature storage was to store the battery in a constant temperature chamber at 60 °C for a total of 56 days. Also, while each secondary battery was stored at 60 °C for 56 days, an amount of gas generation in the secondary battery and open-circuit voltage (OCV) of the secondary battery were observed over time. The OCV is measured using HOIKI (OCV measuring device, model: BT3554). And the volume of gas generated in the secondary battery was calculated using the Archimedes principle.

After 56 days, the OCV deviation (i.e., the degree of OCV drop) and the total volume of gas generated in the secondary battery was calculated. In addition, each secondary battery was charged/discharged at a charge/discharge current density of 0.33C/0.33C, a final charging voltage of 4.2V (NMC/graphite), and a final discharging voltage of 2.5V (NMC/graphite) to measure a battery capacity. And the capacity retention rates of each secondary battery after high-temperature storage were assessed from the measured values. The result is shown in Table 3 below.

**[Table 3]**

| | Type of electrolyte additive used | OCV deviation [mV] | Capacity retention rate [%] | Amount of gas generation [µl] |
|---|---|---|---|---|
| Example 13 Reference | 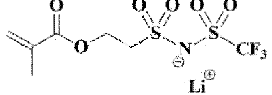 | 33.3 | 98.0 | 1302 |
| Example 14 | 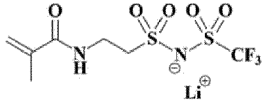 | 33.4 | 97.9 | 1360 |
| Example 15 | 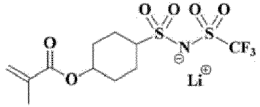 | 33.6 | 97.9 | 1418 |
| Example 16 | 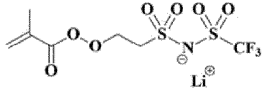 | 34.0 | 97.3 | 1441 |
| Example 17 | 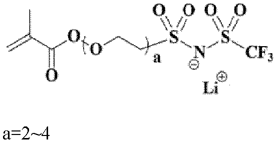 | 33.8 | 97.5 | 1380 |
| Example 18 | 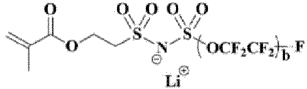 | 34.2 | 97.2 | 1402 |
| Comparative Example 13 | Not added | 38.5 | 95.6 | 2023 |
| Comparative Example 14 | 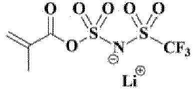 | 40.0 | 95.0 | 1710 |
| Comparative Example 15 | 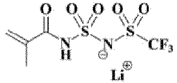 | 39.6 | 95.4 | 1943 |
| Comparative Example 16 | 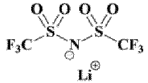 | 38.1 | 95.8 | 1943 |
| Comparative Example 17 | 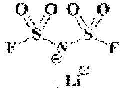 | 39.6 | 96.1 | 1928 |
| Comparative Example 18 | Oligomer originates from <Structural Formula 61> | 39.2 | 95.7 | 1877 |

As shown in Table 3, in the secondary batteries of Examples including the compound represented by Formula 1 as an electrolyte additive according to the present disclosure, it can be seen that, even when being exposed to a high temperature condition, due to the coating film formed on the surfaces of the positive and negative electrodes, the amount of gas generated is significantly reduced by lowering the decomposition of the electrolyte, and the OCV drop phenomenon occurring at the positive electrode decreases.

As above, the present disclosure has been described with reference to exemplary embodiments, but it should be understood by those killed in the art or those of ordinary skill in the art that the present disclosure can be variously modified and changed without departing from the technical scope of the present disclosure described in the accompanying claims.

Accordingly, the technical scope of the present disclosure is not limited to the content described in the detailed description of the specification, but should be defined by the claims.

## Claims

1. An electrolyte composition for a lithium secondary battery, comprising:
a non-aqueous organic solvent;
a lithium salt; and
a compound represented by Formula 1 wherein the compound represented by Formula 1 is one or more compounds selected from Structural Formula 1 to Structural Formula 120 below:
| | | |
|---|---|---|
| Structural Formula 1 | Structural Formula 2 | Structural Formula 3 |
| 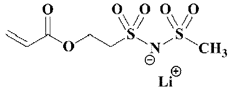 | 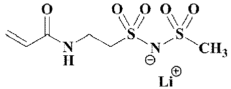 | 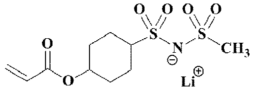 |
| Structural Formula 4 | Structural Formula 5 | Structural Formula 6 |
| 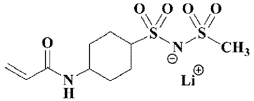 | 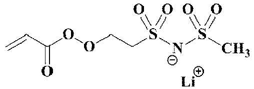 | 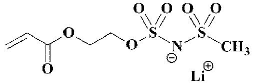 |
| Structural Formula 7 | Structural Formula 8 | Structural Formula 9 |
| 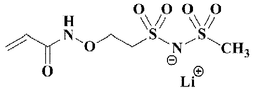 | 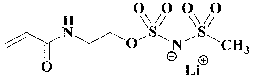 | 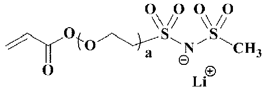 |
| Structural Formula 10 | Structural Formula 11 | Structural Formula 12 |
| 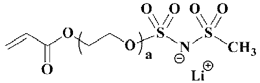 | 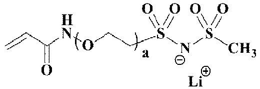 | 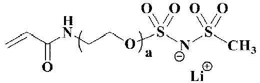 |
| | | |
|---|---|---|
| | Structural Formula 14 | Structural Formula 15 |
| | 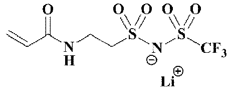 | 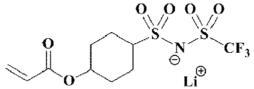 |
| Structural Formula 16 | Structural Formula 17 | Structural Formula 18 |
| 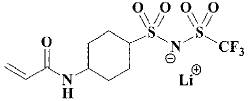 | 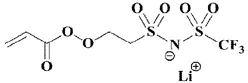 | 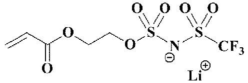 |
| Structural Formula 19 | Structural Formula 20 | Structural Formula 21 |
| 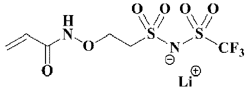 | 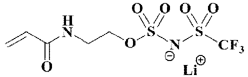 | 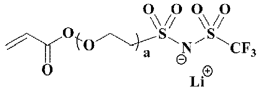 |
| Structural Formula 22 | Structural Formula 23 | Structural Formula 24 |
| 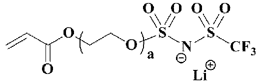 | 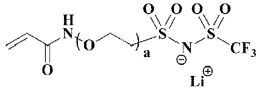 | 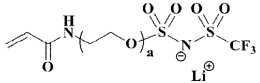 |
| | | |
|---|---|---|
| Structural Formula 25 | Structural Formula 26 | Structural Formula 27 |
| 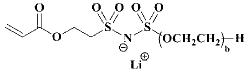 | 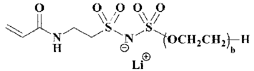 | 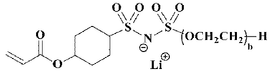 |
| Structural Formula 28 | Structural Formula 29 | Structural Formula 30 |
| 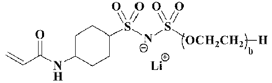 | 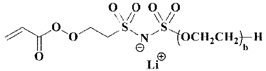 | 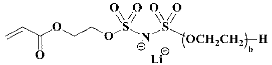 |
| Structural Formula 31 | Structural Formula 32 | Structural Formula 33 |
| 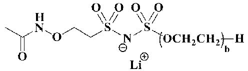 | 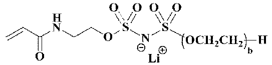 | 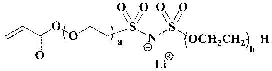 |
| Structural Formula 34 | Structural Formula 35 | Structural Formula 36 |
| 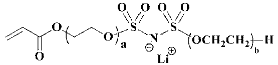 | 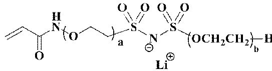 | 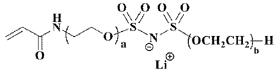 |
| | | |
|---|---|---|
| Structural Formula 37 | Structural Formula 38 | Structural Formula 39 |
| 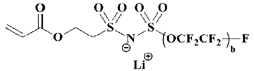 | 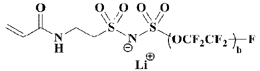 | 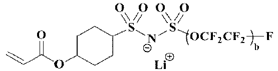 |
| Structural Formula 40 | Structural Formula 41 | Structural Formula 42 |
| 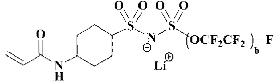 | 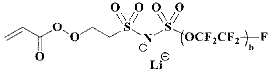 | 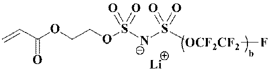 |
| Structural Formula 43 | Structural Formula 44 | Structural Formula 45 |
| 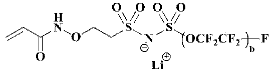 | 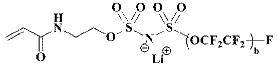 | 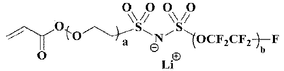 |
| Structural Formula 46 | Structural Formula 47 | Structural Formula 48 |
| 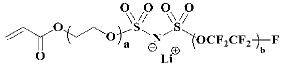 | 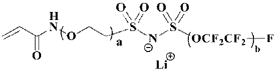 | 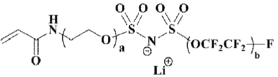 |
| | | |
|---|---|---|
| Structural Formula 49 | Structural Formula 50 | Structural Formula 51 |
| 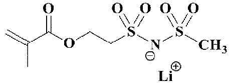 | 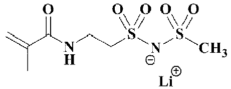 | 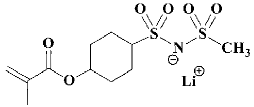 |
| Structural Formula 52 | Structural Formula 53 | Structural Formula 54 |
| 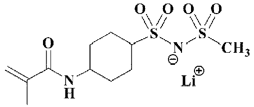 | 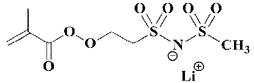 | 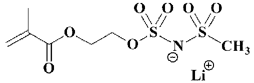 |
| Structural Formula 55 | Structural Formula 56 | Structural Formula 57 |
| 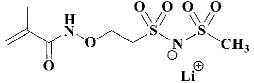 | 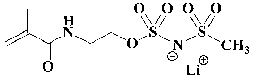 | 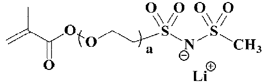 |
| Structural Formula 58 | Structural Formula 59 | Structural Formula 60 |
| 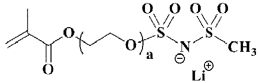 | 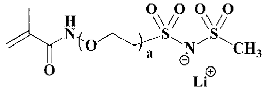 | 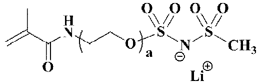 |
| | | |
|---|---|---|
| | Structural Formula 62 | Structural Formula 63 |
| | 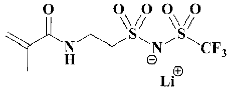 | 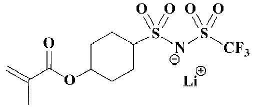 |
| Structural Formula 64 | Structural Formula 65 | Structural Formula 66 |
| 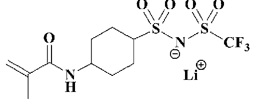 | 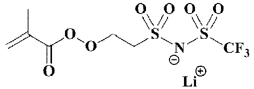 | 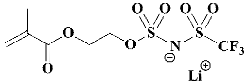 |
| Structural Formula 67 | Structural Formula 68 | Structural Formula 69 |
| 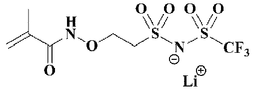 | 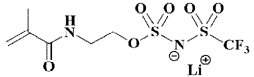 | 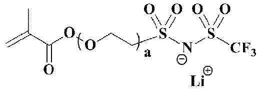 |
| Structural Formula 70 | Structural Formula 71 | Structural Formula 72 |
| 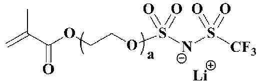 | 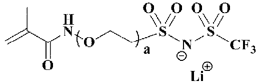 | 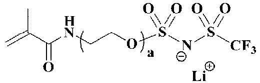 |
| | | |
|---|---|---|
| Structural Formula 73 | Structural Formula 74 | Structural Formula 75 |
| 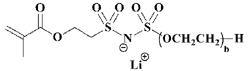 | 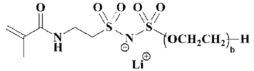 | 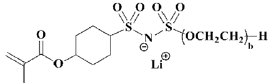 |
| Structural Formula 76 | Structural Formula 77 | Structural Formula 78 |
| 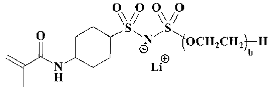 | 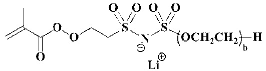 | 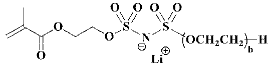 |
| Structural Formula 79 | Structural Formula 80 | Structural Formula 81 |
| 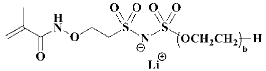 | 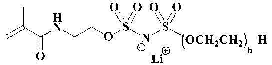 | 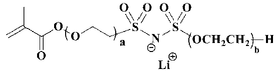 |
| Structural Formula 82 | Structural Formula 83 | Structural Formula 84 |
| 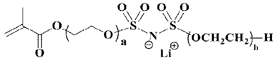 | 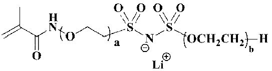 | 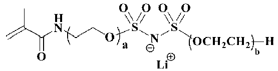 |
| | | |
|---|---|---|
| Structural Formula 85 | Structural Formula 86 | Structural Formula 87 |
| 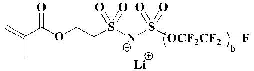 | 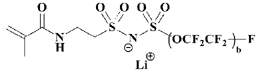 | 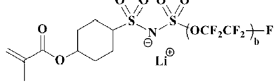 |
| Structural Formula 88 | Structural Formula 89 | Structural Formula 90 |
| 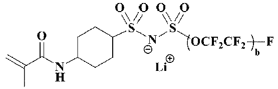 | 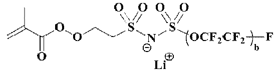 | 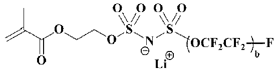 |
| Structural Formula 91 | Structural Formula 92 | Structural Formula 93 |
| 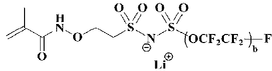 | 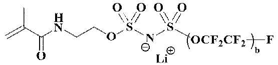 | 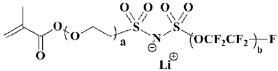 |
| Structural Formula 94 | Structural Formula 95 | Structural Formula 96 |
| 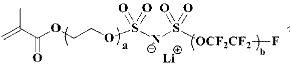 | 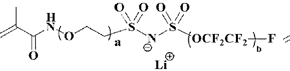 | 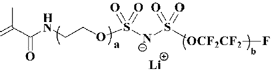 |
| | | |
|---|---|---|
| Structural Formula 97 | Structural Formula 98 | Structural Formula 99 |
| 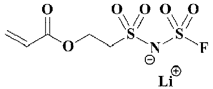 | 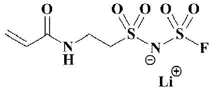 | 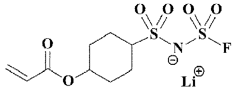 |
| Structural Formula 100 | Structural Formula 101 | Structural Formula 102 |
| 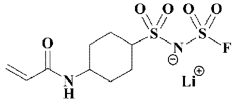 | 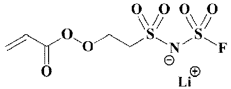 | 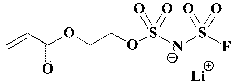 |
| Structural Formula 103 | Structural Formula 104 | Structural Formula 105 |
| 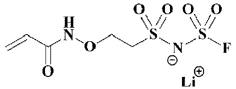 | 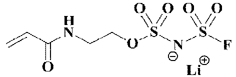 | 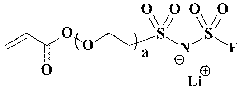 |
| Structural Formula 106 | Structural Formula 107 | Structural Formula 108 |
| 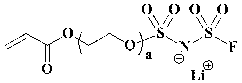 | 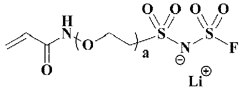 | 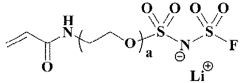 |
| | | |
|---|---|---|
| Structural Formula 109 | Structural Formula 110 | Structural Formula 111 |
| 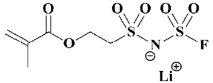 | 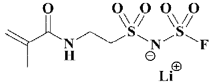 | 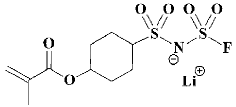 |
| Structural Formula 112 | Structural Formula 113 | Structural Formula 114 |
| 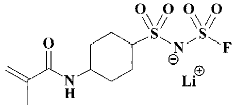 | 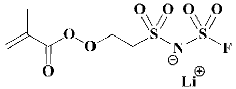 | 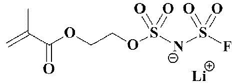 |
| Structural Formula 115 | Structural Formula 116 | Structural Formula 117 |
| 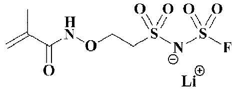 | 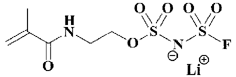 | 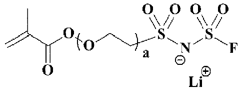 |
| Structural Formula 118 | Structural Formula 119 | Structural Formula 120 |
| 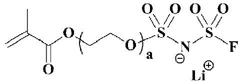 | 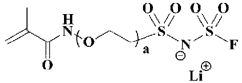 | 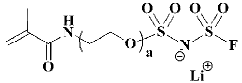 |
wherein, in Structural Formula 9~12, 21~48, 57~60, 69~96, 105~108, and 117~120, a and b is an integer of 2 to 4 respectively.

2. The electrolyte composition of claim 1, wherein the compound represented by Formula 1 is present in an amount of 0.01 to 3 wt% with respect to the total weight of the electrolyte composition.

3. The electrolyte composition of claim 1, wherein the lithium salt comprises one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

4. The electrolyte composition of claim 1, wherein the non-aqueous organic solvent comprises one or more selected from the group consisting of N-methyl-2-pyrrolidinone, ethylene carbonate, propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, a dioxolane derivative, sulfolane, methyl sulfolane, 1,3-dimethyl-2-imidazolidinone, a propylene carbonate derivative, a tetrahydrofuran derivative, ether, methyl propionate, and ethyl propionate.

5. A lithium secondary battery, comprising:
an electrode assembly having a positive electrode, a negative electrode, and a separator interposed between the positive electrode and the negative electrode, and
an electrolyte composition for a lithium secondary battery,
wherein the positive electrode comprises one or more positive electrode active materials of lithium metal oxides represented by Formulas 2 and 3:
[Formula 2] Liₓ[Ni_{y}Co_{z}Mn_{w}M¹ᵥ]O₂
[Formula 3] LiM²ₚMn₍₂₋ₚ₎O₄
In Formulas 2 and 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6, wherein the electrolyte composition for a lithium secondary battery comprises a non-aqueous organic solvent;
a lithium salt; and
a compound represented by Formula 1: 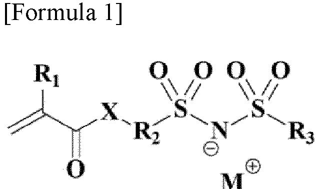
wherein,
R₁ is hydrogen or an alkyl group having 1 to 4 carbon atoms,
R₂ includes one or more selected from an alkylene group having 1 to 10 carbon atoms, an oxyalkylene group having 1 to 10 carbon atoms, a cycloalkylene group having 5 to 10 carbon atoms, and 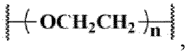
R₃ is a fluoro group, an alkyl group having 1 to 10 carbon atoms, an alkoxy group having 1 to 10 carbon atoms, or 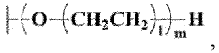
wherein one or more hydrogens included in the alkyl groups, the alkylene group, the alkoxy group, the oxyalkylene group, the cycloalkylene group, 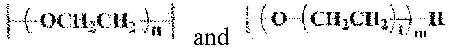 are optionally substituted with a fluorine atom,
X is an oxygen atom (O) or -NR₄, R₄ is hydrogen or an alkyl group having 1 to 4 carbon atoms,
M comprises one or more selected from the group consisting of one or more selected from the group consisting of lithium, sodium, potassium, tetraalkyl ammonium having 1 to 4 carbon atoms, and tetraalkyl phosphonium having 1 to 4 carbon atoms,
1 is an integer of 1 to 6, and
each of m and n is an integer of 2 to 20.

6. The secondary battery of claim 5, wherein the positive electrode active material comprises one or more selected from the group consisting of LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂, and LiNi_{0.5}Mn_{1.5}O₄.

7. The secondary battery of claim 5, wherein the negative electrode active material consists of a carbon material and a silicon material, and
the silicon material comprises one or more of silicon (Si), silicon carbide (SiC) and silicon oxide (SiO_{q}, 0.8≤q≤2.5).

8. The secondary battery of claim 7, wherein the silicon material is present in an amount of 1 to 20 wt% with respect to the total weight of the negative electrode active material.

9. The secondary battery of claim 5, wherein in Formula 1, R₁ is hydrogen or a methyl group,
R₂ comprises one or more selected from a methylene group, an ethylene group, a propylene group, an oxymethylene group, an oxyethylene group, an oxypropylene group, a cyclobutylene group, a cyclopentylene group, a cyclohexylene group, a cycloheptylene group, 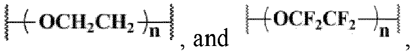
R₃ is a fluoro group, a methyl group, an ethyl group, a propyl group, a methoxy group, an ethoxy group, 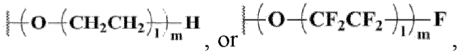
X is an oxygen atom (O), -NH, or -NCH₃,
M is lithium,
l is an integer of 1 or 2, and
each of m and n is an integer of 2 to 10.

10. The secondary battery of claim 5, comprising the electrolyte composition of claims 1-4.

## Patentansprüche

1. Elektrolytzusammensetzung für eine Lithium-Sekundärbatterie, umfassend:
ein nichtwässriges organisches Lösungsmittel;
ein Lithiumsalz; und
eine Verbindung, dargestellt durch die Formel 1, wobei die durch Formel 1 dargestellte Verbindung eine oder mehrere Verbindungen ist, die ausgewählt sind aus den folgenden Strukturformeln 1 bis 120:
| | | |
|---|---|---|
| Strukturformel 1 | Strukturformel 2 | Strukturformel 3 |
| 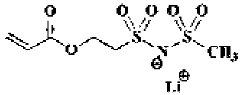 | 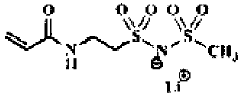 | 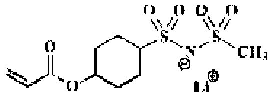 |
| Strukturformel 4 | Strukturformel 5 | Strukturformel 6 |
| 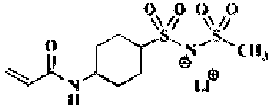 | 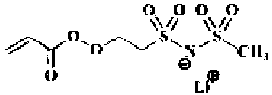 | 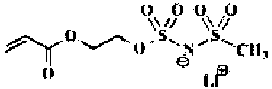 |
| Strukturformel 7 | Strukturformel 8 | Strukturformel 9 |
| 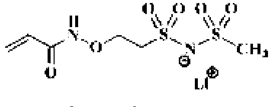 | 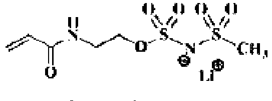 | 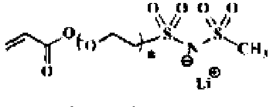 |
| Strukturformel 10 | Strukturformel 11 | Strukturformel 12 |
| 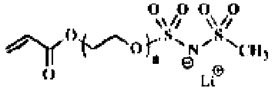 | 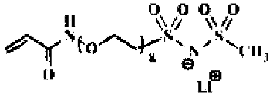 | 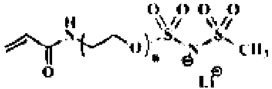 |
| | Strukturformel 14 | Strukturformel 15 |
| | 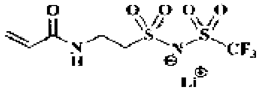 | 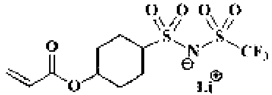 |
| Strukturformel 16 | Strukturformel 17 | Strukturformel 18 |
| 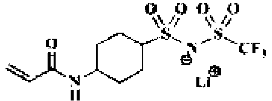 | 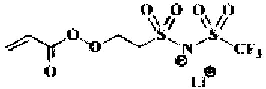 | 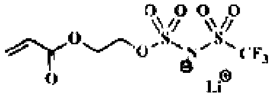 |
| Strukturformel 19 | Strukturformel 20 | Strukturformel 21 |
| 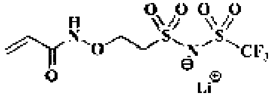 | 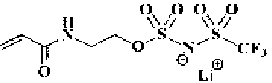 | 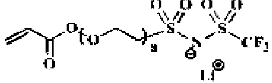 |
| Strukturformel 22 | Strukturformel 23 | Strukturformel 24 |
| 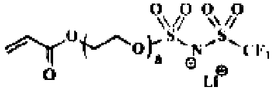 | 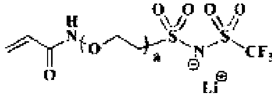 | 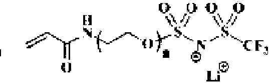 |
| Strukturformel 25 | Strukturformel 26 | Strukturformel 27 |
| 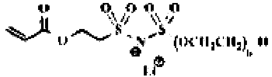 | 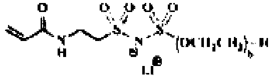 | 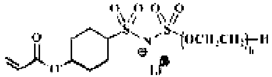 |
| Strukturformel 28 | Strukturformel 29 | Strukturformel 30 |
| 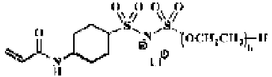 | 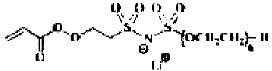 | 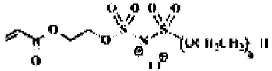 |
| Strukturformel 31 | Strukturformel 32 | Strukturformel 33 |
| 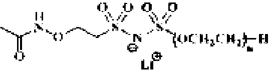 | 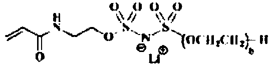 | 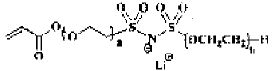 |
| Strukturformel 34 | Strukturformel 35 | Strukturformel 36 |
| 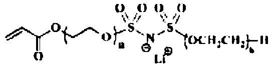 | 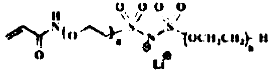 | 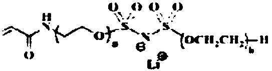 |
| Strukturformel 37 | Strukturformel 38 | Strukturformel 39 |
| 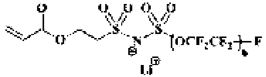 | 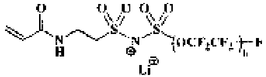 | 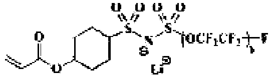 |
| Strukturformel 40 | Strukturformel 41 | Strukturformel 42 |
| 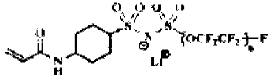 | 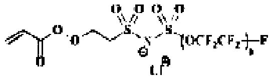 | 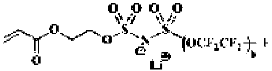 |
| Strukturformel 43 | Strukturformel 44 | Strukturformel 45 |
| 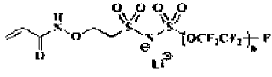 | 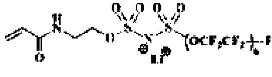 | 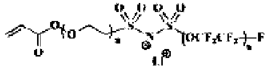 |
| Strukturformel 46 | Strukturformel 47 | Strukturformel 48 |
| 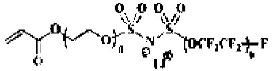 | 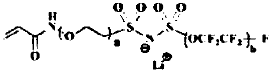 | 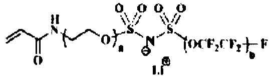 |
| Strukturformel 49 | Strukturformel 50 | Strukturformel 51 |
| 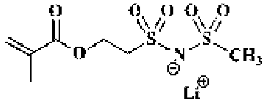 | 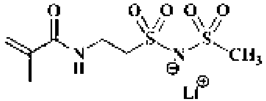 | 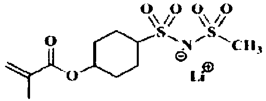 |
| Strukturformel 52 | Strukturformel 53 | Strukturformel 54 |
| 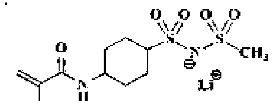 | 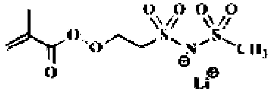 | 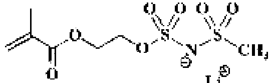 |
| Strukturformel 55 | Strukturformel 56 | Strukturformel 57 |
| 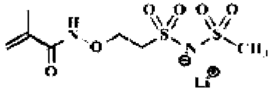 | 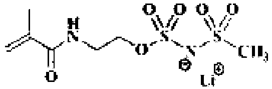 | 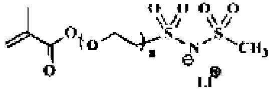 |
| Strukturformel 58 | Strukturformel 59 | Strukturformel 60 |
| 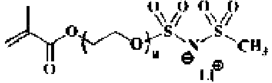 | 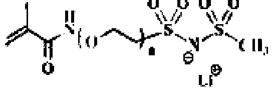 | 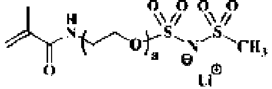 |
| | Strukturformel 62 | Strukturformel 63 |
| | 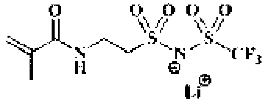 | 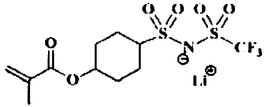 |
| Strukturformel 64 | Strukturformel 65 | Strukturformel 66 |
| 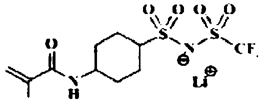 | 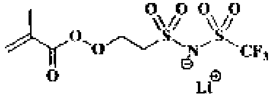 | 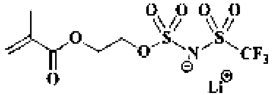 |
| Strukturformel 67 | Strukturformel 68 | Strukturformel 69 |
| 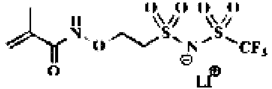 | 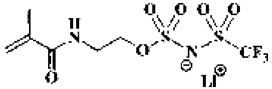 | 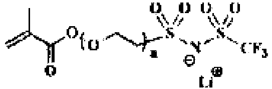 |
| Strukturformel 70 | Strukturformel 71 | Strukturformel 72 |
| 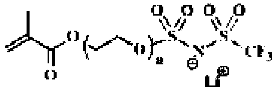 | 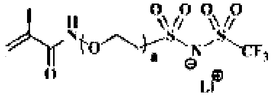 | 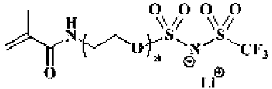 |
| Strukturformel 73 | Strukturformel 74 | Strukturformel 75 |
| 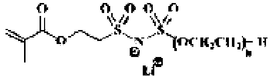 | 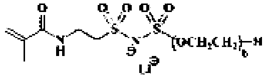 | 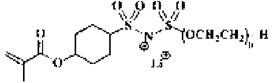 |
| Strukturformel 76 | Strukturformel 77 | Strukturformel 78 |
| 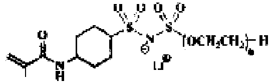 | 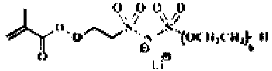 | 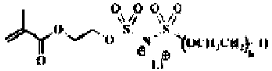 |
| Strukturformel 79 | Strukturformel 80 | Strukturformel 81 |
| 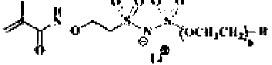 | 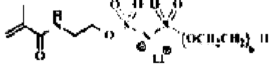 | 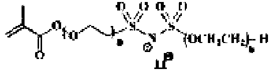 |
| Strukturformel 82 | Strukturformel 83 | Strukturformel 84 |
| 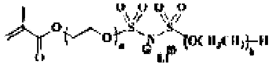 | 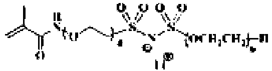 | 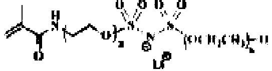 |
| Strukturformel 85 | Strukturformel 86 | Strukturformel 87 |
| 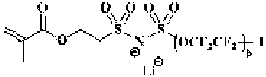 | 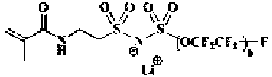 | 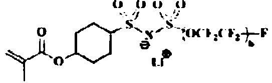 |
| Strukturformel 88 | Strukturformel 89 | Strukturformel 90 |
| 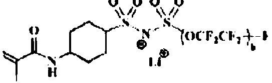 | 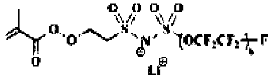 | 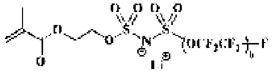 |
| Strukturformel 91 | Strukturformel 92 | Strukturformel 93 |
| 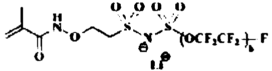 | 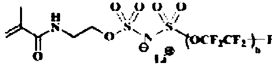 | 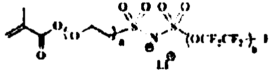 |
| Strukturformel 94 | Strukturformel 95 | Strukturformel 96 |
| 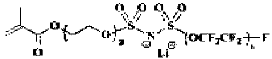 | 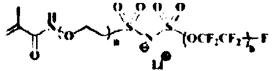 | 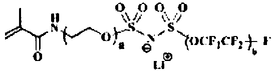 |
| Strukturformel 97 | Strukturformel 98 | Strukturformel 99 |
| 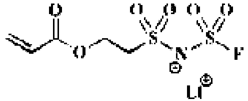 | 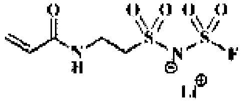 | 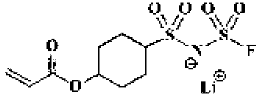 |
| Strukturformel 100 | Strukturformel 101 | Strukturformel 102 |
| 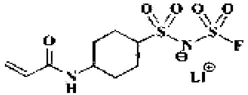 | 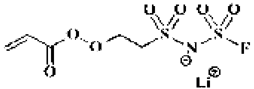 | 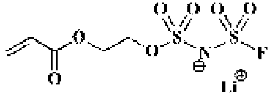 |
| Strukturformel 103 | Strukturformel 104 | Strukturformel 105 |
| 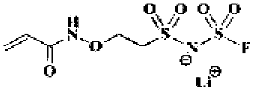 | 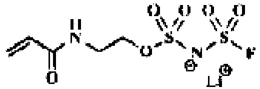 | 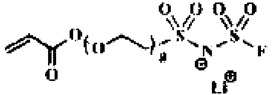 |
| Strukturformel 106 | Strukturformel 107 | Strukturformel 108 |
| 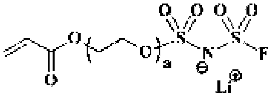 | 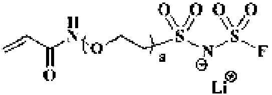 | 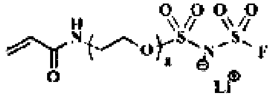 |
| Strukturformel 109 | Strukturformel 110 | Strukturformel 111 |
| 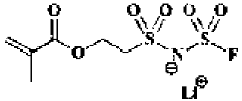 | 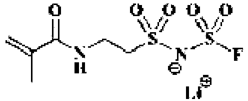 | 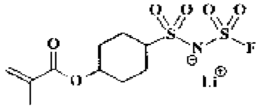 |
| Strukturformel 112 | Strukturformel 113 | Strukturformel 114 |
| 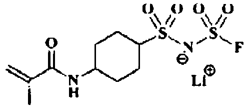 | 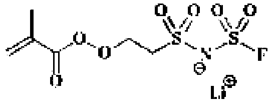 | 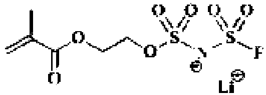 |
| Strukturformel 115 | Strukturformel 116 | Strukturformel 117 |
| 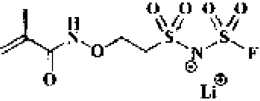 | 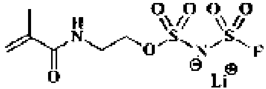 | 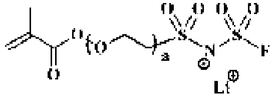 |
| Strukturformel 118 | Strukturformel 119 | Strukturformel 120 |
| 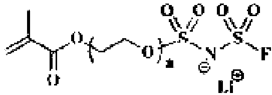 | 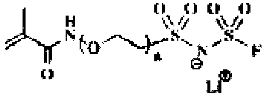 | 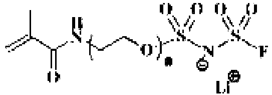 |
worin in den Strukturformeln 9-12, 21-48, 57-60, 69-96, 105-108 und 117-120 a und b jeweils eine ganze Zahl von 2 bis 4 sind.

2. Elektrolytzusammensetzung gemäß Anspruch 1, wobei die durch Formel 1 dargestellte Verbindung in einer Menge von 0,01 bis 3 Gew.-%, bezogen auf das Gesamtgewicht der Elektrolytzusammensetzung, vorhanden ist.

3. Elektrolytzusammensetzung gemäß Anspruch 1, wobei das Lithiumsalz eines oder mehrere umfasst, die ausgewählt sind aus der Gruppe bestehend aus LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi und (FSO₂)₂NLi.

4. Elektrolytzusammensetzung gemäß Anspruch 1, wobei das nichtwässrige organische Lösungsmittel eines oder mehrere umfasst, die ausgewählt sind aus der Gruppe bestehend aus N-Methyl-2-pyrrolidinon, Ethylencarbonat, Propylencarbonat, Butylencarbonat, Dimethylcarbonat, Diethylcarbonat, γ-Butyrolacton, 1,2-Dimethyoxyethan, Tetrahydrofuran, 2-Methyltetrahydrofuran, Dimethylsulfoxid, 1,3-Dioxolan, Formamid, Dimethylformamid, Dioxolan, Acetonitril, Nitromethan, Methylformiat, Methylcitrat, Phosphorsäuretriester, Trimethoxymethan, einem Dioxolanderivat, Sulfolan, Methylsulfolan, 1,3-Dimethyl-2-imidazolidinon, einem Propylencarbonatderivat, einem Tetrahydrofuranderivat, Ether, Methylpropionat und Ethylpropionat.

5. Lithium-Sekundärbatterie, umfassend:
eine Elektrodenanordnung mit einer positiven Elektrode, einer negativen Elektrode und einem zwischen der positiven Elektrode und der negativen Elektrode angeordneten Separator, und
eine Elektrolytzusammensetzung für eine Lithium-Sekundärbatterie,
wobei die positive Elektrode ein oder mehrere Positivelektrodenaktivmaterialien aus Lithiummetalloxiden umfasst, die dargestellt sind durch die Formeln 2 und 3:
[Formel 2] Liₓ[Ni_{y}Co_{z}Mn_{w}M¹ᵥ]O₂
[Formel 3] LiM²ₚMn₍₂₋ₚ₎O₄
worin in den Formeln 2 und 3
M¹ ist ein oder mehrere Elemente, die ausgewählt sind aus der Gruppe bestehend aus W, Cu, Fe, V, Cr, Ti, Zr, Zn, Al, In, Ta, Y, La, Sr, Ga, Sc, Gd, Sm, Ca, Ce, Nb, Mg, B und Mo,
x, y, z, w und v erfüllen jeweils 1,0 ≤ x ≤ 1,30, 0,5 ≤ y < 1, 0 < z ≤ 0,3, 0 < w ≤ 0,3 und 0 ≤ v ≤ 0,1, wobei y + z + w + v = 1 ist,
M² ist Ni, Co oder Fe und
p ist 0,05 ≤ p ≤ 0,6, wobei die Elektrolytzusammensetzung für eine Lithium-Sekundärbatterie umfasst
ein nichtwässriges organisches Lösungsmittel,
ein Lithiumsalz und
eine Verbindung der Formel 1: 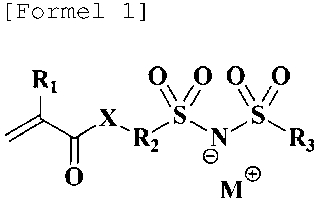
worin
R₁ Wasserstoff oder eine Alkylgruppe mit 1 bis 4 Kohlenstoffatomen ist,
R₂ eines oder mehrere umfasst, die ausgewählt sind aus einer Alkylengruppe mit 1 bis 10 Kohlenstoffatomen, einer Oxyalkylengruppe mit 1 bis 10 Kohlenstoffatomen, einer Cycloalkylengruppe mit 5 bis 10 Kohlenstoffatomen und 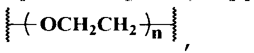
R₃ eine Fluorgruppe, eine Alkylgruppe mit 1 bis 10 Kohlenstoffatomen, eine Alkoxygruppe mit 1 bis 10 Kohlenstoffatomen oder 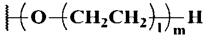 ist,
worin ein oder mehrere Wasserstoffatome, die in den Alkylgruppen, der Alkylengruppe, der Alkoxygruppe, der Oxyalkylengruppe, der Cycloalkylengruppe 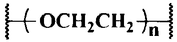 und 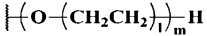 enthalten sind, optional durch ein Fluoratom substituiert sind,
X ein Sauerstoffatom (O) oder -NR₄ ist, worin R₄ Wasserstoff oder eine Alkylgruppe mit 1 bis 4 Kohlenstoffatomen ist,
M eines oder mehrere umfasst, die ausgewählt sind aus der Gruppe bestehend aus einem oder mehreren, die ausgewählt sind aus der Gruppe bestehend aus Lithium, Natrium, Kalium, Tetraalkylammonium mit 1 bis 4 Kohlenstoffatomen und Tetraalkylphosphonium mit 1 bis 4 Kohlenstoffatomen,
1 eine ganze Zahl von 1 bis 6 ist, und
m und n jeweils eine ganze Zahl von 2 bis 20 sind.

6. Sekundärbatterie gemäß Anspruch 5, wobei das Positivelektrodenaktivmaterial eines oder mehrere umfasst, die ausgewählt sind aus der Gruppe bestehend aus LiNi_{0,8}Co_{0,1}Mn_{0,1}O₂, LiNi_{0,6}CO_{0,2}Mn_{0,2}O₂, LiNi_{0,9}CO_{0,05}Mn_{0,05}O₂, LiNi_{0,6}Co_{0,2}Mn_{0,1}Al_{0,1}O₂, LiNi_{0,6}CO_{0,2}Mn_{0,15}sAl_{0,05}O₂, LiNi_{0,7}CO_{0,1}Mn_{0,1}Al_{0,1}O₂ und LiNi_{0,5}Mn_{1,5}O₄.

7. Sekundärbatterie gemäß Anspruch 5, wobei das Negativelektrodenaktivmaterial aus einem Kohlenstoffmaterial und einem Siliziummaterial besteht und
das Siliziummaterial eines oder mehrere umfasst aus Silizium (Si), Siliziumkarbid (SiC) und Siliziumoxid (SiO_{q}, 0,8 ≤ q ≤ 2,5).

8. Sekundärbatterie gemäß Anspruch 7, wobei das Siliziummaterial in einer Menge von 1 bis 20 Gew.-%, bezogen auf das Gesamtgewicht des Negativelektrodenaktivmaterials, vorhanden ist.

9. Sekundärbatterie gemäß Anspruch 5, wobei in Formel 1 R₁ Wasserstoff oder eine Methylgruppe ist,
R₂ eines oder mehrere umfasst aus einer Methylengruppe, einer Ethylengruppe, einer Propylengruppe, einer Oxymethylengruppe, einer Oxyethylengruppe, einer Oxypropylengruppe, einer Cyclobutylengruppe, einer Cyclopentylengruppe, einer Cyclohexylengruppe, einer Cycloheptylengruppe, 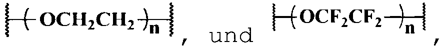
R₃ eine Fluorgruppe, eine Methylgruppe, eine Ethylgruppe, eine Propylgruppe, eine Methoxygruppe, eine Ethoxygruppe, 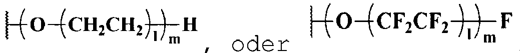 ist,
X ein Sauerstoffatom (O), -NH oder -NCH₃ ist,
M Lithium ist,
l eine ganze Zahl von 1 oder 2 ist und
m und n jeweils eine ganze Zahl von 2 bis 10 sind.

10. Sekundärbatterie gemäß Anspruch 5, umfassend die Elektrolytzusammensetzung gemäß mindestens einem der Ansprüche 1 bis 4.

## Revendications

1. Composition d'électrolyte pour une batterie secondaire au lithium, comprenant :
un solvant organique non aqueux ;
un sel de lithium ; et
un composé représenté par la formule 1, dans laquelle le composé représenté par la formule 1 est un ou plusieurs composés choisis parmi la formule développée 1 à la formule développée 120 ci-dessous :
| | | |
|---|---|---|
| Formule développée 1 | Formule développée 2 | Formule développée 3 |
| 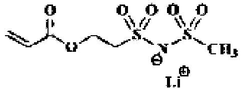 | 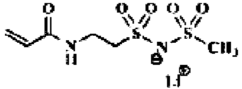 | 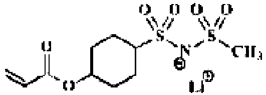 |
| Formule développée 4 | Formule développée 5 | Formule développée 6 |
| 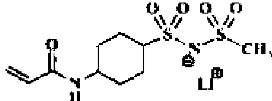 | 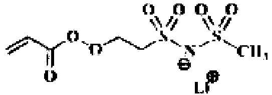 | 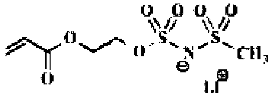 |
| Formule développée 7 | Formule développée 8 | Formule développée 9 |
| 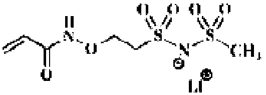 | 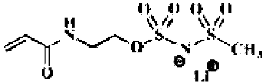 | 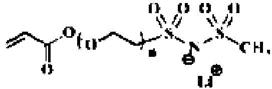 |
| Formule développée 10 | Formule développée 11 | Formule développée 12 |
| 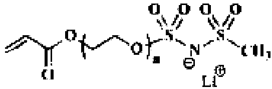 | 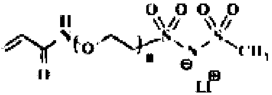 | 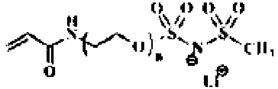 |
| | | |
|---|---|---|
| | Formule développée 14 | Formule développée 15 |
| | 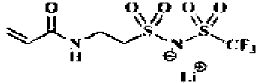 | 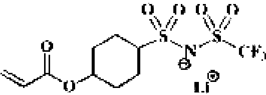 |
| Formule développée 16 | Formule développée 17 | Formule développée 18 |
| 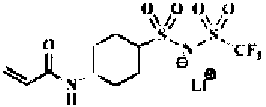 | 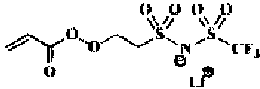 | 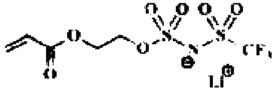 |
| Formule développée 19 | Formule développée 20 | Formule développée 21 |
| 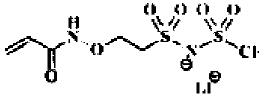 | 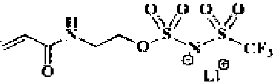 | 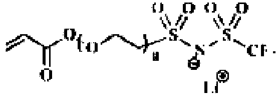 |
| Formule développée 22 | Formule développée 23 | Formule développée 24 |
| 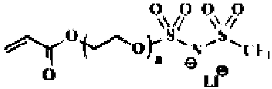 | 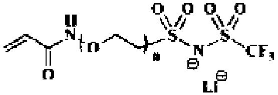 | 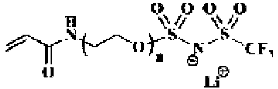 |
| | | |
|---|---|---|
| Formule développée 25 | Formule développée 26 | Formule développée 27 |
| 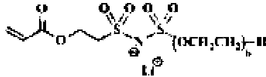 | 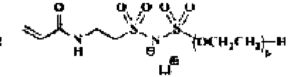 | 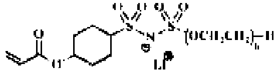 |
| Formule développée 28 | Formule développée 29 | Formule développée 30 |
| 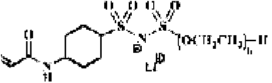 | 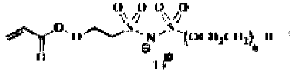 | 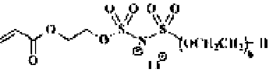 |
| Formule développée 31 | Formule développée 32 | Formule développée 33 |
| 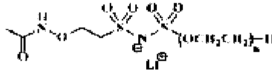 | 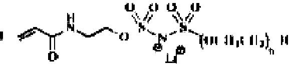 | 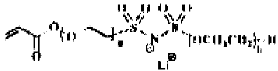 |
| Formule développée 34 | Formule développée 35 | Formule développée 36 |
| 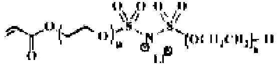 | 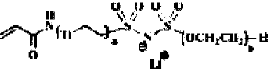 | 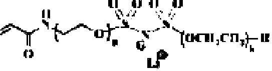 |
| | | |
|---|---|---|
| Formule développée 37 | Formule développée 38 | Formule développée 39 |
| 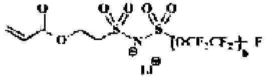 | 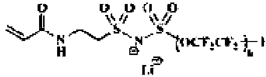 | 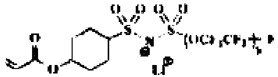 |
| Formule développée 40 | Formule développée 41 | Formule développée 42 |
| 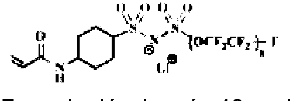 | 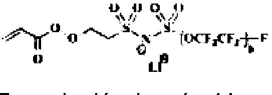 | 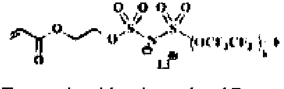 |
| Formule développée 43 | Formule développée 44 | Formule développée 45 |
| 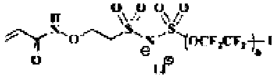 | 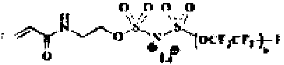 | 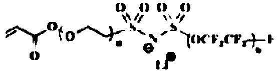 |
| Formule développée 46 | Formule développée 47 | Formule développée 48 |
| 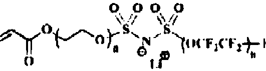 | 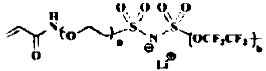 | 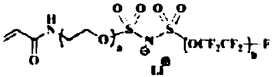 |
| Formule développée 49 | Formule développée 50 | Formule développée 51 |
| 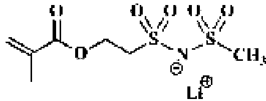 | 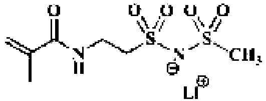 | 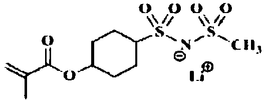 |
| Formule développée 52 | Formule développée 53 | Formule développée 54 |
| 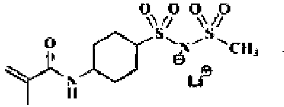 | 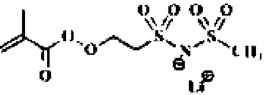 | 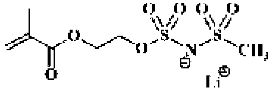 |
| Formule développée 55 | Formule développée 56 | Formule développée 57 |
| 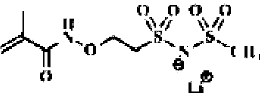 | 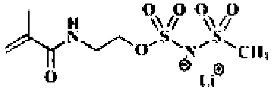 | 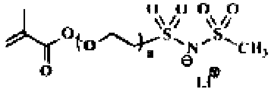 |
| Formule développée 58 | Formule développée 59 | Formule développée 60 |
| 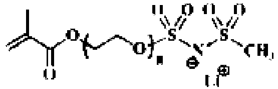 | 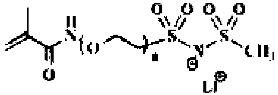 | 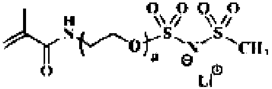 |
| | | |
|---|---|---|
| | Formule développée 62 | Formule développée 63 |
| | 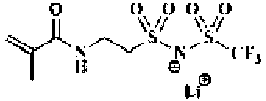 | 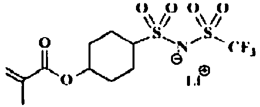 |
| Formule développée 64 | Formule développée 65 | Formule développée 66 |
| 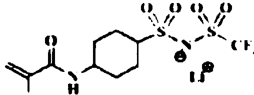 | 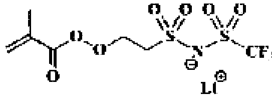 | 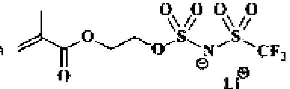 |
| Formule développée 67 | Formule développée 68 | Formule développée 69 |
| 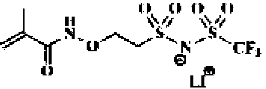 | 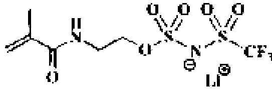 | 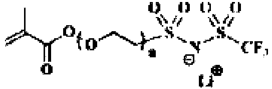 |
| Formule développée 70 | Formule développée 71 | Formule développée 72 |
| 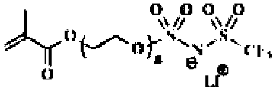 | 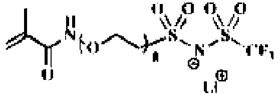 | 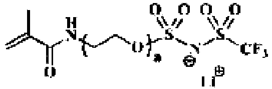 |
| Formule développée 73 | Formule développée 74 | Formule développée 75 |
| 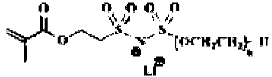 | 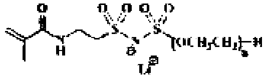 | 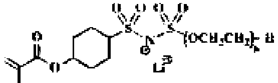 |
| Formule développée 76 | Formule développée 77 | Formule développée 78 |
| 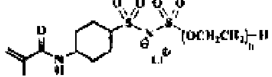 | 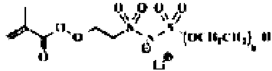 | 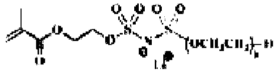 |
| Formule développée 79 | Formule développée 80 | Formule développée 81 |
| 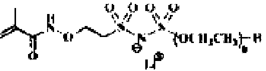 | 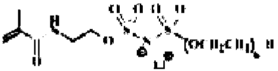 | 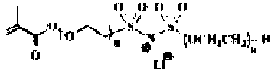 |
| Formule développée 82 | Formule développée 83 | Formule développée 84 |
| 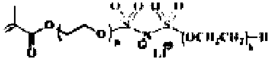 | 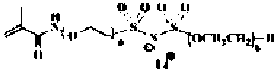 | 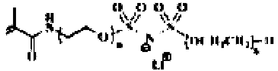 |
| | | |
|---|---|---|
| Formule développée 85 | Formule développée 86 | Formule développée 87 |
| 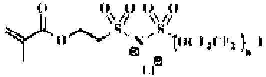 | 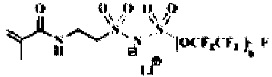 | 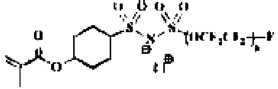 |
| Formule développée 88 | Formule développée 89 | Formule développée 90 |
| 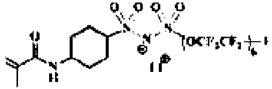 | 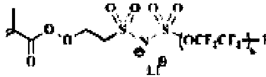 | 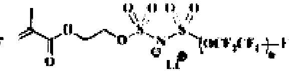 |
| Formule développée 91 | Formule développée 92 | Formule développée 93 |
| 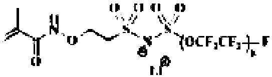 | 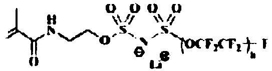 | 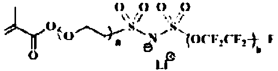 |
| Formule développée 94 | Formule développée 95 | Formule développée 96 |
| 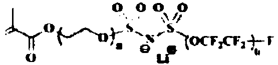 | 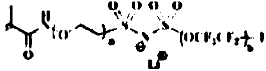 | 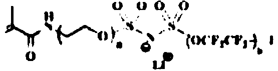 |
| Formule développée 97 | Formule développée 98 | Formule développée 99 |
| 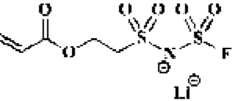 | 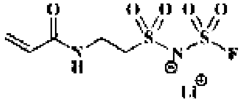 | 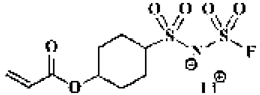 |
| Formule développée 100 | Formule développée 101 | Formule développée 102 |
| 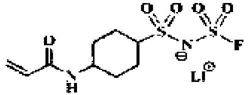 | 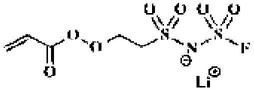 | 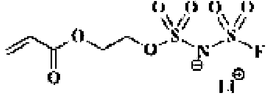 |
| Formule développée 103 | Formule développée 104 | Formule développée 105 |
| 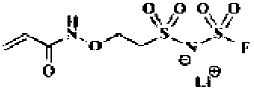 | 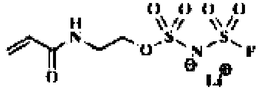 | 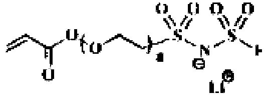 |
| Formule développée 106 | Formule développée 107 | Formule développée 108 |
| 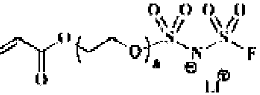 | 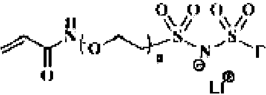 | 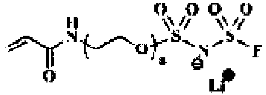 |
| | | |
|---|---|---|
| Formule développée 109 | Formule développée 110 | Formule développée 111 |
| 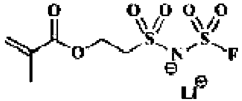 | 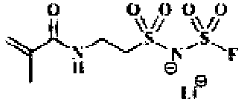 | 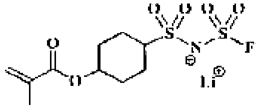 |
| Formule développée 112 | Formule développée 113 | Formule développée 114 |
| 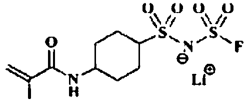 | 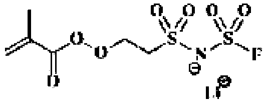 | 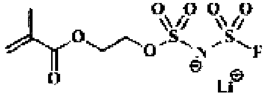 |
| Formule développée 115 | Formule développée 116 | Formule développée 117 |
| 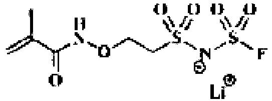 | 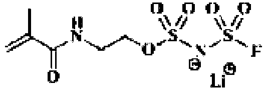 | 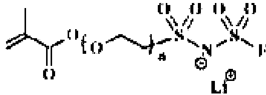 |
| Formule développée 118 | Formule développée 119 | Formule développée 120 |
| 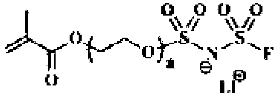 | 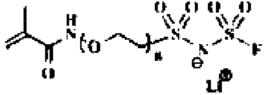 | 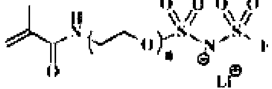 |
dans laquelle, dans les formules développées 9-12, 21-48, 57-60, 69-96, 105-108 et 117-120, a et b sont un nombre entier de 2 à 4 respectivement.

2. Composition d'électrolyte selon la revendication 1, dans laquelle le composé représenté par la formule 1 est présent en une quantité de 0,01 à 3 % en poids par rapport au poids total de la composition d'électrolyte.

3. Composition d'électrolyte selon la revendication 1, dans laquelle le sel de lithium comprend un ou plusieurs éléments choisis dans le groupe consistant en LiCl, LiBr, Lil, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi et (FSO₂)₂NLi.

4. Composition d'électrolyte selon la revendication 1, dans laquelle le solvant organique non aqueux comprend un ou plusieurs éléments choisis dans le groupe consistant en N-méthyl-2-pyrrolidinone, carbonate d'éthylène, carbonate de propylène, carbonate de butylène, carbonate de diméthyle, carbonate de diéthyle, γ-butyrolactone, 1,2-diméthoxyéthane, tétrahydrofurane, 2-méthyl-tétrahydrofurane, diméthylsulfoxyde, 1,3-dioxolane, formamide, diméthylformamide, dioxolane, acétonitrile, nitrométhane, formiate de méthyle, citrate de méthyle, triester d'acide phosphorique, triméthoxyméthane, un dérivé de dioxolane, sulfolane, méthylsulfolane, 1,3-diméthyl-2-imidazolidinone, un dérivé de carbonate de propylène, un dérivé de tétrahydrofurane, éther, propionate de méthyle et propionate d'éthyle.

5. Batterie secondaire au lithium, comprenant :
un ensemble d'électrodes présentant une électrode positive, une électrode négative et un séparateur interposé entre l'électrode positive et l'électrode négative, et
une composition d'électrolyte pour une batterie secondaire au lithium,
dans laquelle l'électrode positive comprend un ou plusieurs matériaux actifs d'électrode positive d'oxydes de métal de lithium représentés par les formules 2 et 3 :
[Formule 2] Liₓ[Ni_{y}Co_{z}Mn_{w}M¹ᵥ]O₂
[Formule 3] LiM²ₚMn₍₂₋ₚ₎O₄
dans les formules 2 et 3,
M¹ est un ou plusieurs éléments choisis dans le groupe consistant en W, Cu, Fe, V, Cr, Ti, Zr, Zn, Al, In, Ta, Y, La, Sr, Ga, Sc, Gd, Sm, Ca, Ce, Nb, Mg, B et Mo,
x, y, z, w et v satisfont respectivement 1,0 ≤ x ≤ 1,30, 0,5 ≤ y < 1, 0 < z ≤ 0,3, 0 < w ≤ 0,3 et 0 ≤ v ≤ 0,1, dans laquelle y + z + w + v = 1,
M² est Ni, Co ou Fe, et
p est tel que 0,05 ≤ p ≤ 0,6, dans laquelle la composition d'électrolyte pour une batterie secondaire au lithium comprend un solvant organique non aqueux ;
un sel de lithium ; et
un composé représenté par la formule 1 : 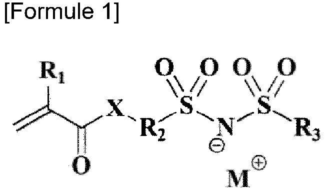
dans laquelle,
R₁ est hydrogène ou un groupe alkyle présentant de 1 à 4 atomes de carbone,
R₂ inclut un ou plusieurs éléments choisis dans un groupe alkylène présentant de 1 à 10 atomes de carbone, un groupe oxyalkylène présentant de 1 à 10 atomes de carbone, un groupe cycloalkylène présentant de 5 à 10 atomes de carbone, et 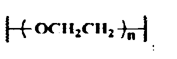
R₃ est un groupe fluoro, un groupe alkyle présentant de 1 à 10 atomes de carbone, un groupe alcoxy présentant de 1 à 10 atomes de carbone, ou 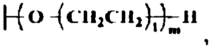
dans laquelle un ou plusieurs hydrogènes inclus dans les groupes alkyle, le groupe alkylène, le groupe alcoxy, le groupe oxyalkylène, le groupe cycloalkylène, 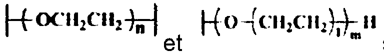 sont facultativement substitués par un atome de fluor,
X est un atome d'oxygène (O) ou -NR₄, R₄ est hydrogène ou un groupe alkyle présentant de 1 à 4 atomes de carbone,
M comprend un ou plusieurs éléments choisis dans le groupe consistant en lithium, sodium, potassium, tétralkylammonium présentant de 1 à 4 atomes de carbone et tétralkylphosphonium présentant de 1 à 4 atomes de carbone,
1 est un nombre entier de 1 à 6, et
chacun de m et n est un nombre entier de 2 à 20.

6. Batterie secondaire selon la revendication 5, dans laquelle le matériau actif d'électrode positive comprend un ou plusieurs éléments choisis dans le groupe consistant en LiNi_{0,8}Co_{0,1}Mn_{0,1}O₂, LiNi_{0,6}Co_{0,2}Mn_{0,2}O₂, LiNi_{0,9}Co_{0,05}Mn_{0,05}O₂, LiNi_{0,6}Co_{0,2}Mn_{0,1}Al_{0,1}O₂, LiNi_{0,6}Co_{0,2}Mn_{0,15}Al_{0,05}O₂, LiNi_{0,7}Co_{0,1}Mn_{0,1}Al_{0,1}O₂ et LiNi_{0,5}Mn_{1,5}O₄.

7. Batterie secondaire selon la revendication 5, dans laquelle le matériau actif d'électrode négative consiste en un matériau carboné et un matériau en silicium, et
le matériau en silicium comprend un ou plusieurs éléments choisis parmi le silicium (Si), le carbure de silicium (SiC) et l'oxyde de silicium (SiO_{q}, 0,8 ≤ q ≤ 2,5).

8. Batterie secondaire selon la revendication 7, dans laquelle le matériau en silicium est présent en une quantité de 1 à 20 % en poids par rapport au poids total du matériau actif d'électrode négative.

9. Batterie secondaire selon la revendication 5, dans laquelle, dans la formule 1, R₁ est un hydrogène ou un groupe méthyle,
R₂ comprend un ou plusieurs éléments choisis parmi un groupe méthylène, un groupe éthylène, un groupe propylène, un groupe oxyméthylène, un groupe oxyéthylène, un groupe oxypropylène, un groupe cyclobutylène, un groupe cyclopentylène, un groupe cyclohexylène, un groupe cycloheptylène, 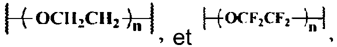
R₃ est un groupe fluoro, un groupe méthyle, un groupe éthyle, un groupe propyle, un groupe méthoxy, un groupe éthoxy, 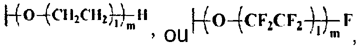
X est un atome d'oxygène (O), -NH ou -NCH₃,
M est lithium,
1 est un nombre entier de 1 ou 2, et
chacun de m et n est un nombre entier de 2 à 10.

10. Batterie secondaire selon la revendication 5, comprenant la composition d'électrolyte selon les revendications 1 à 4.
